# EUROPEAN PATENT APPLICATION

(11) **EP 4 803 103 A1**
(43) Date of publication of application: **09.09.2026**
(21) Application number: 25161523.3
(22) Date of filing: 04.03.2025
(51) Int. Cl.: A61K 47/64, A61K 47/68

(54) **NEW ANTIBODY-DRUG CONJUGATES COMPRISING PSAR UNITS**

(71) Applicant: Adcytherix SAS, 13007 Marseille (FR)
(72) Inventor: MASSIOT, Julien, 13007 Marseille (FR); PREVILLE, Xavier, 13007 Marseille (FR); ELANDS, Jack, 13007 Marseille (FR)
(74) Representative: Weickmann & Weickmann PartmbB

(57) **Abstract**

The present invention relates to linker-drug conjugates and linker compounds with a polysarcosine side-chain, corresponding antibody-drug conjugates, methods for preparing such antibody-drug conjugates as well as pharmaceutical compositions comprising the antibody drug-conjugates and their use in medicine.

## Description

The present invention relates to linker-drug conjugates and linker compounds, corresponding antibody-drug conjugates, methods for preparing such antibody-drug conjugates as well as pharmaceutical compositions comprising the antibody drug-conjugates and their use in medicine.

Antibody-Drug Conjugates (ADCs) represent an emerging class of therapeutic agents that combine the specificity of monoclonal antibodies with the potency of cytotoxic drugs. ADCs are designed to selectively deliver cytotoxic agents to cancer cells, thereby minimizing the systemic toxicity often associated with conventional chemotherapy.

In the design of an ADC, next to the antibody and the drug or payload, the choice of the linker connecting the two components is crucial. Physicochemical properties of the ADC can be tuned via the linker, and it has been shown that linker chemistry affects toxicity, stability and potency of ADCs (Baah et al. 2021, Molecules 26(10), 2943).

An appropriate linker should convey high plasma stability to the ADC and prevent premature release of the cytotoxic agent, also called payload, which would cause off-target toxicity. Conversely, at the target site, the linker must allow for release of the payload. For non-cleavable linkers, payload release may happen through complete degradation of the antibody in the lysosome. In these cases, the released agent may comprise the payload itself, the linker and an amino acid from the antibody.

More complete and specific release of the payload may be achieved through use of cleavable linkers capable of undergoing specific cleavage reactions at a designated cleavage site. To ensure plasma stability, the cleavage reaction should rely on a trigger, preferably a trigger linked to the tumor environment and/or the tumor cells. For example, chemically cleavable linkers include acid cleavable linkers which hydrolyze in the lysosome or the acidic tumor microenvironment, or reduction sensitive linkers which may be cleaved by reaction with glutathione in the cytoplasm of target cells. Among the most promising cleavable linkers are enzymatically cleavable linkers depending on enzymes having high expression levels in tumors, such as cathepsin-cleavable linkers or glycosidase-cleavable linkers.

In view of the hydrophobicity of most cytotoxic agents used in ADCs, an important further function of the linker is to increase hydrophilicity of the ADC. Increasing hydrophilicity and shielding hydrophobicity of ADCs has been shown to prevent aggregation, to reduce nonspecific clearance and off-target toxicity and to improve efficacy of the ADCs. Hydrophilicity of linkers may be enhanced by the incorporation of polyethylene glycol (PEG) or polysarcosine (PSAR) units, with the latter providing improved biocompatibility and biodegradability properties.

As the field of ADCs is expanding through exploration of new target antigens and payloads, a need remains for versatile linker structures which can be used with various payloads and antibodies and are easy to synthesize and conjugate.

In particular, such linker structures should provide good stability and hydrophilicity of the ADCs to maximize their effectiveness and should preferably allow for specific and efficient release of the drug at the target site.

This task is surprisingly solved by the present invention, which provides a modular linker structure that can easily be adapted and tuned for use with different payloads and/or antibodies. The linker structure affords high hydrophilicity and stability and may be provided with various drug-release mechanisms depending on the desired use. Further, linker-drug conjugates according to the invention may be provided through straightforward synthesis as shown herein below.

### LINKER-DRUG CONJUGATE

In a first aspect, the present invention relates to a linker-drug conjugate of formula (I), or a pharmaceutically acceptable salt thereof, 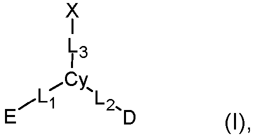 wherein
- Cy: is a 5- or 6-membered saturated, unsaturated or partially unsaturated heterocycle comprising 1-3 nitrogen atoms,
- D: is a drug or an active agent,
- E: is -NH-C(=O)-(C₁-C₁₀-alkylene)-Q,
- Q: is an antibody coupling group or a leaving group,
- X: is 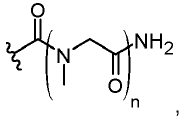
- L₁: is a linker comprising 1-12 consecutive ethylene glycol units and/or a peptide spacer comprising 1-8 amino acids,
- L₂: is 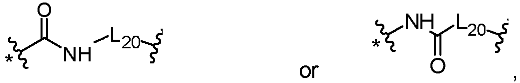
- L₂₀: is a linker comprising one or more units selected from
- one or more C₁-C₁₀-alkylene units,
- a triazole unit having the formula 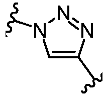
- a peptide comprising 2-4 amino acids, and
- a self-immolative spacer, particularly a self-immolative spacer with an enzyme-cleavable trigger moiety,
- L₃: is a bond or a linker independently defined as L₁, and
- n: is an integer from 1-100, preferably 5-20, more preferably 8-12,
wherein * indicates the bond to Cy.

Cy is an organic heterocycle, i.e., a heterocyclic compound wherein the cycle consists of carbon atoms and at least one non-carbon atom, i.e. a heteroatom. Cy comprises 1, 2 or 3 nitrogen atoms but may additionally comprise further heteroatoms such as oxygen or sulfur, preferably oxygen. In a preferred embodiment, all heteroatoms in Cy are nitrogen atoms.

In a preferred embodiment, Cy is a fully saturated heterocycle. In an alternative embodiment, Cy is an aromatic heterocycle.

In a particularly preferred embodiment, Cy is selected from the group consisting of a pyrrolidine unit, a piperazine unit, an imidazole unit, a pyrazole unit, a 1,2,4-triazole unit and a 1,2,3-triazole unit. A pyrrolidine unit or a piperazine unit are particularly preferred.

In a particular embodiment, the linker-drug conjugate of the invention is represented by formula (I) as defined above, wherein the partial structure represented by formula (la), 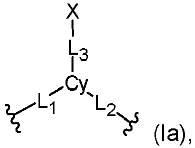 is

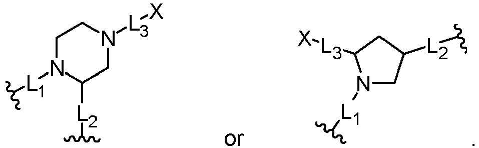

The linker-drug conjugate of the invention comprises a polysarcosine (PSAR) unit X as defined above, connected to Cy via L₃. In a preferred embodiment, n, which represents the number of repeats of polysarcosine units in X, is an integer from 1-100, preferably 5-20, more preferably 8-12, in particular 10.

PSAR units of defined lengths may be provided by solid-phase synthesis as described in the examples below. The hydrophilicity of the linker-drug conjugate is enhanced by the presence of polysarcosine units and may easily be modulated by tuning the number of polysarcosine repeats. As PSAR is not only highly hydrophilic and water-soluble, but also biodegradable and non-immunogenic, it provides an efficient and safe way of shielding hydrophobicity e.g. of a drug.

According to the invention, group E comprising an antibody-coupling group or leaving group Q is linked via a linker L₁ to Cy.

Group E is -NH-C(=O)-(C₁-C₁₀-alkylene)-Q, preferably -NH-C(=O)-(C₁-C₅-alkylene)-Q, more preferably -NH-C(=O)-(C₁-C₃-alkylene)-Q and in particular -NH-C(=O)-(CH₂)₂-Q. Preferably, the alkylene group in E is a saturated linear alkylene group.

In preferred embodiments, Q is an antibody-coupling group, preferably a thiol-reactive group, more preferably a maleimide group. Preferably, if Q is a maleimide group, the nitrogen atom of Q is covalently linked to the C₁-C₁₀-alkylene group in E.

In an alternative embodiment, the antibody-coupling group Q is an aldehyde-reactive moiety suitable for Hydrazino-iso-Pictet-Spengler (HIPS) chemistry. HIPS ligation to aldehydes introduced into an antibody may be conducted under mild reaction conditions and lead to formation of a stable C-C bond.

In a further preferred embodiment, Q is a leaving group, preferably a leaving group selected from Br and I.

Preferably, the linker L₁ is covalently bound to a nitrogen atom in Cy, preferably via a peptide bond.

It is preferred that L₁ comprises up to 12 ethylene glycol units.

In preferred embodiments, L₁ is a linker comprising 1-12 consecutive ethylene glycol units, preferably 1-10, more preferably 1-4 consecutive ethylene glycol units, most preferably 2 consecutive ethylene glycol units, with ethylene glycol units having the general formula

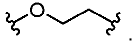

L₁ may comprise one or more groups of consecutive ethylene glycol units, preferably 1-3 groups, more preferably 1-2 groups, particularly one group of consecutive ethylene glycol units. If L₁ comprises more than one group of consecutive ethylene glycol units, it is preferred that the total number of ethylene glycol units in L₁ is 2-12, and each group comprises at least one ethylene glycol unit. If two groups of ethylene glycol units are directly adjacent to each other, they may preferably be connected by an amide bond.

In addition to the length of linker L₁ and therefore the distance in the molecule of the antibody-coupling group Q to the central heterocycle Cy and the drug D, the number of ethylene glycol units in L₁ also affects hydrophilicity of the linker-drug conjugate.

In preferred embodiments, L₁ is 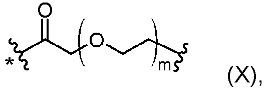
wherein m is an integer from 1-12, preferably 1-10, more preferably 1-8, more preferably 1-6, even more preferably 1-4, particularly 2, and
wherein * indicates the bond to Cy, preferably to a nitrogen atom in Cy.

In further preferred embodiments, L₁ is a linker comprising a peptide spacer comprising 1-8 amino acids, preferably 1-4 amino acids. In still further preferred embodiments, L₁ comprises 1-12 consecutive ethylene glycol units and a peptide spacer comprising 1-4 amino acids.

For any peptide comprised in L₁ or L₂₀ in formula (I), amino acids may be selected independently. The amino acids are α-amino acids and may independently be L or D amino acids. In a preferred embodiment, at least one amino acid is an L amino acid. In other preferred embodiments all amino acids are D or L amino acids, preferably L amino acids. Amino acids may independently be unnatural or natural, proteinogenic or non-proteinogenic amino acids. For L₁, natural proteinogenic amino acids are preferred.

L₃ links the polysarcosine unit X to the heterocycle Cy. Preferably, L₃ is covalently bound to a nitrogen atom or a carbon atom in Cy.

In preferred embodiments, L₃ is a covalent bond between X and Cy, preferably a covalent bond between X and a nitrogen atom or a carbon atom in Cy.

In further preferred embodiments, L₃ is a linker independently defined as L₁. In this context, "independently defined" means that L₃ is a linker with a structure as defined above for L₁, but in the same molecule, L₁ and L₃ are chosen independently according to this definition.

Preferably, in the same molecule, i.e. in a linker-drug conjugate of the invention as defined above, or a compound of the invention or ADC of the invention as defined below, L₁ corresponds to a preferred embodiment of L₁ as defined above, and L₃ is either a bond or a linker having the same structure as L₁ or corresponding to a different preferred embodiment as defined for L₁.

The drug D of the linker-drug conjugate of the invention is linked to Cy via a linker L₂ of the formula 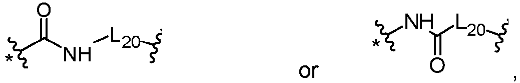 wherein L₂₀ is a linker comprising one or more units selected from
- one or more C₁-C₁₀-alkylene units,
- a triazole unit having the formula 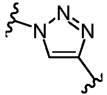
- a peptide comprising 2-4 amino acids, and
- a self-immolative spacer, particularly a self-immolative spacer with an enzyme-cleavable trigger moiety, and
wherein * indicates the bond to Cy.

Preferably, L₂ is covalently bound to a carbon atom in Cy.

In a preferred embodiment, L₂₀ is a C₁-C₁₀-alkylene, preferably a C₁-C₈₋alkylene, more preferably a C₁-C₆₋alkylene and even more preferably a C₁-C₄₋alkylene. The alkylene is preferably a linear, saturated and unbranched alkylene.

In particularly preferred embodiments, L₂₀ comprises or is 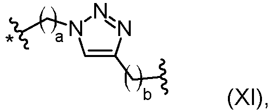 wherein a and b are independently selected integers from 1-8, and * indicates the bond closer to Cy. Preferably, a is an integer from 1-5, more preferably 1-3, particularly 3, and b is an integer from 1-5 more preferably 1-2, particularly 1.

In a preferred embodiment, L₂₀ consists of the triazole structure of formula (XI), wherein a and b are as defined above. In another preferred embodiment, L₂₀ comprises the triazole structure of formula (XI), wherein a and b are as defined above. In this case, it is preferred that the bond in the triazole structure above indicated with * is a covalent bond to the peptide group in L₂ which connects L₂₀ with Cy.

In embodiments wherein L₂₀ comprises the triazole structure of formula (XI) as defined above, the triazole unit may be generated through reactions known in the art, preferably through a copper-catalyzed azide-alkyne cycloaddition as known in the art and as described in the Examples.

In preferred embodiments, L₂₀ comprises or is a peptide comprising 2-4 amino acids, wherein the N terminus of the peptide is preferably closer to Cy and the C terminus is preferably closer to D. The peptide may consist of any natural or unnatural, proteinogenic or non-proteinogenic, D or L α-amino acids as defined above. It is preferred that the N and C termini of the peptide are connected to the remaining molecule via peptide bonds.

Preferably, the peptide is sensitive to cleavage by a lysosomal peptidase. Several lysosomal peptidases including cathepsin and legumain have been reported to be highly expressed in cancer cells. When using the linker-drug conjugate of the invention in an ADC for the treatment of cancer, it is preferred that a peptide comprised in L₂₀ is cleavable by such a lysosomal peptidase that is highly expressed in cancer cells, preferably by cathepsin or legumain. This approach enhances specificity of drug release and may therefore reduce off-target toxicity. Cathepsin-cleavable peptides are particularly preferred and are known in the art.

The cleavage site of the peptidase-cleavable peptide, particularly of the cathepsin-cleavable peptide, is preferably at the C terminus of the peptide, which is preferably the part of the peptide closer to D as defined above. In a preferred embodiment, the C terminus of the cleavable peptide is linked to D directly, via a short spacer or via a self-immolative spacer as will be described below.

In a particular embodiment, L₂₀ comprises a peptide selected from valine-citrulline (Val-Cit), Val-Ala, cyclobutane-1,1-dicarboxamide (cBu)-Cit and GGFG. These peptides are sensitive to cathepsin cleavage, particularly at the C terminus. Particularly preferred sequences are Val-Cit and GGFG.

In preferred embodiments, L₂₀ comprises a self-immolative spacer. The self-immolative spacer may be part of an activatable self-immolative group comprised in L₂₀. A "self-immolative group" as used herein is a part of a linker which may be used in an antibody-drug conjugate with a function to conditionally release a free drug at the site targeted by the ligand unit. The activatable self-immolative group preferably comprises an activatable group or trigger moiety and a self-immolative spacer unit. Upon activation of the activatable group, for example by enzymatic conversion of an amide group to an amino group, by reduction of a disulfide to a free thiol group or by enzymatic cleavage of the activatable group, a self-immolative reaction sequence is initiated that leads to release of the free drug by one or more of various mechanisms. Preferably, the self-immolative spacer is eliminated in the self-immolative reaction sequence.

Preferably, at least a part of the self-immolative spacer in L₂₀ is an integral part in the linker L₂, i.e., Cy and D are linked via at least a part of the self-immolative spacer and potential other groups. A trigger moiety attached to the self-immolative spacer may or may not be an integral part of the linker L₂ as defined above. In a preferred embodiment, the trigger moiety is not an integral part of the linker L₂ but branches off from the self-immolative spacer.

If a self-immolative spacer is present in L₂₀, it is preferably covalently bound to the drug D, in such a way that upon self-immolation, the linkage between Cy and D is interrupted and the drug D is released. In this case, it is further preferred that the drug D is released in a traceless manner, i.e., that it retains no remaining residues of the self-immolative spacer or of L₂₀ upon release. In this way, optimal efficacy of the released drug is ensured.

The self-immolative spacer preferably comprised in L₂₀ may particularly be selected from 1,6-elimination spacers, such as para-aminobenzyl carbamate (PABC) and derivatives thereof or 1,6-elimination spacers based on 4-hydroxybenzyl alcohol, 3-nitrobenzyl alcohol or 2-azidobenzyl alcohol, disulfide-based self-immolative spacers, such as disulfide-linked dithiophenol spacers, and cyclization-driven self-immolative spacers, such as maleic anhydride derivatives.

In a particularly preferred embodiment, L₂₀ comprises a self-immolative spacer comprising a *para-N-* or *O*-substituted benzyl carbamate unit. For example, L₂₀ may comprise a para-aminobenzyl carbamate (PABC) unit or a para-alkoxybenzyl carbamate unit. The benzyl moiety may comprise no further substituent, or it may comprise one further substituent, preferably in meta position. In a preferred embodiment, the para-*N*- or *O*-substituted benzyl carbamate unit further comprises a nitro group in *meta* position. As used herein, *para* and *meta* are indicated relative to the benzylic carbon of the benzyl group.

Preferably, the self-immolative spacer is covalently bound to D. In a preferred embodiment, the self-immolative spacer is covalently bound to the nitrogen of a primary or secondary amine group in D. It is particularly preferred that the self-immolative spacer comprises a *para-N-* or *O*-substituted benzyl carbamate unit wherein the nitrogen of the carbamate is the nitrogen of a primary or secondary amine group in D. The linkage to the remaining part of L₂₀ or L₂ may in this case be mediated via further substitution of the benzylic carbon or via the *para-N-* or *O-*substituent.

In a preferred embodiment, L₂₀ comprises a self-immolative spacer with an enzyme-cleavable trigger moiety. As used herein, an enzyme-cleavable trigger moiety is a moiety which may be cleaved off of the remaining molecule by the action of an enzyme present in a mammalian organism, preferably in a human organism. For example, the enzyme may be a peptidase, glycosidase or sulfatase. It is preferred that the enzyme is highly expressed in tumor cells. It is particularly preferred that the enzyme is present and preferably abundant in a tumor or tumor microenvironment, particularly in the extracellular space and/or in the cytosol and/or endolysosomal system of tumor cells.

The enzyme-cleavable trigger moiety is preferably bound to the self-immolative spacer such that its presence inhibits self-immolation of the spacer and its cleavage triggers self-immolation of the spacer.

The enzyme-cleavable trigger moiety may be a dipeptide as described above, which is preferably an integral part of linker L₂. In particular embodiments, the enzyme-cleavable trigger moiety is a sugar or a sulfate and is more preferably β-D-glucuronic acid, β-D-galactose or a sulfate group. Preferably, the sugar is bound to the self-immolative spacer via a glycosidic bond and is cleavable by a glycosidase. For example, the β-glucuronide trigger moiety is cleavable by β-glucuronidase, and a β-galactoside trigger moiety is cleavable by β-galactosidase. Both of these enzymes are highly expressed in tumors. Analogously, a sulfate trigger moiety may be cleaved by sulfatase, which was also found to be highly expressed in tumor cells.

In particular embodiments, L₂₀ comprises a self-immolative spacer corresponding to formula (XII), 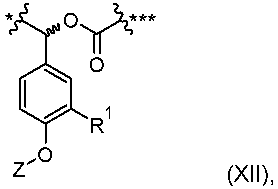 wherein
- Z: is an enzyme-cleavable trigger moiety preferably selected from 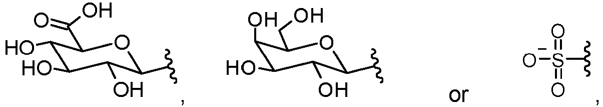 preferably wherein Z is 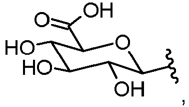
- R¹: is NO₂ or H, preferably NO₂,
- *: indicates the bond closer to Cy, and
- ***: indicates the bond closer to D, preferably a bond to D, more preferably a bond to the nitrogen of a primary or secondary amine group in D.

In particularly preferred embodiments, L₂₀ comprises and preferably corresponds to a structure of formula (XIII): 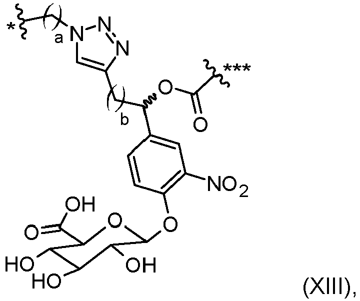 wherein
- a: is an integer from 1-8, preferably 1-5, more preferably 3,
- b: is an integer from 1-8, preferably 1-5, more preferably 1,
- *: indicates the bond closer to Cy, and
- ***: indicates the bond to D, preferably the bond to the nitrogen of a primary or secondary amine group in D.

In the tumor environment or in tumor cells, the β-glucuronide trigger moiety in the above structure may be cleaved by β-glucuronidase, leading to elimination of the self-immolative PABC-analogous unit and release of D.

In further preferred embodiments, L₂₀ comprises a self-immolative spacer structure according to formula (XIV), preferably in combination with a trigger moiety which is an integral part of L₂₀, such as a peptidase-cleavable peptide as defined above: 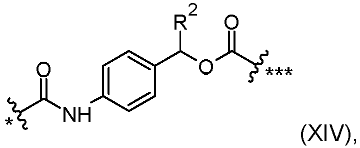 wherein
- R²: is H, R²⁰ or C(=O)R²⁰, preferably H or C(=O)R²⁰, more preferably H,
- R²⁰: is selected from C₁-C₂₄ (hetero)alkyl groups, C₃-C₁₀ (hetero)cycloalkyl groups, C₂-C₁₀ (hetero)aryl groups, C₃-C₁₀ alkyl(hetero)aryl groups and C₃-C₁₀ (hetero)arylalkyl groups, which are optionally substituted and optionally interrupted by one or more heteroatoms selected from O, S and NR²¹ with R²¹ being independently selected from H or C₁-C₄ alkyl groups, preferably 4-methyl-piperazine or morpholine, and
* indicates the bond closer to Cy, and *** indicates the bond closer to D, preferably a bond to D, more preferably a bond to the nitrogen of a primary or secondary amine group in D.

In a particular embodiment, L₂₀ comprises a peptide as defined above for L₂₀ and a self-immolative spacer as defined above. It is particularly preferred that L₂₀ consists of said peptide and said self-immolative spacer. It is further preferred that the peptide is a dipeptide selected from Val-Cit, Val-Ala, and cBu-Cit and the self-immolative spacer is a PABC spacer as defined in formula (XIV), wherein the peptidic carbonyl is the C-terminal carbonyl of the dipeptide. In such embodiments, the peptide may be seen as cathepsin-sensitive trigger moiety for the self-immolative PABC spacer. Upon cleavage of the peptide, PABC may undergo an elimination reaction, preferably leading to traceless release of D.

In further preferred embodiments, L₂₀ comprises a structure of formula (XV): 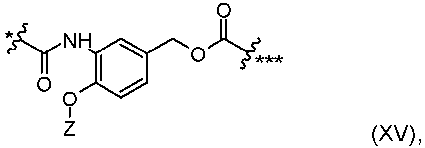 wherein Z is an enzyme-cleavable trigger moiety and is preferably 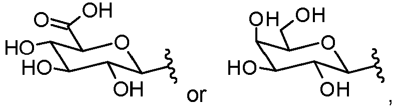 more preferably 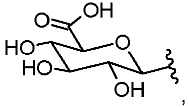 and wherein * indicates the bond closer to Cy, and *** indicates the bond closer to D, preferably a bond to D, more preferably a bond to the nitrogen of a primary or secondary amine group in D.

In the above embodiment, upon cleavage of the trigger moiety in formula (XV), e.g. cleavage of the β-glucuronide by β-glucuronidase, the remaining spacer may undergo self-immolation and release the drug D which is directly or indirectly, preferably directly bound to the site marked with ***.

The drug D may be any drug or cytotoxic agent suitable for antibody-drug conjugates. In a preferred embodiment, the drug D of the linker-drug conjugate of the invention is a drug or active agent bearing a reactive amine. In preferred embodiments, the drug D is exatecan.

In particularly preferred embodiments, the linker-drug conjugate of the invention corresponds to any one of formulas (II) or (III), 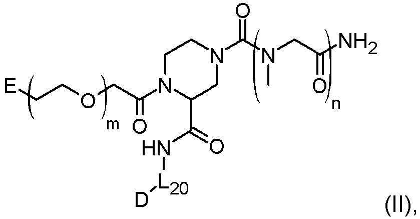 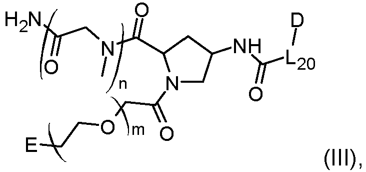 wherein
- D: is a drug or an active agent,
- E: is -NH-C(=O)-(C₁-C₃-alkylene)-Q,
- Q: is an antibody coupling group, preferably a maleimide group,
- L₂₀: is a linker selected from
a C₁-C₁₀-alkylene, 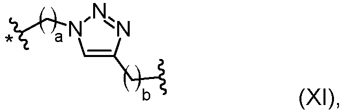 and 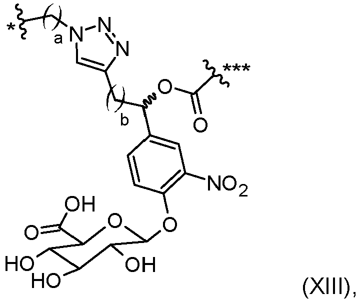
- a: is an integer from 1-8, preferably 1-5, more preferably 1-3,
- b: is an integer from 1-8, preferably 1-5, more preferably 1-2,
- m: is an integer from 1-8, preferably 1-6, more preferably 1-4,
- n: is an integer from 5-20, preferably 8-12, more preferably 10,
- *: indicates the bond closer to Cy, and
- ***: indicates the bond to D, preferably the bond to the nitrogen of a primary or secondary amine group in D.

In the above embodiment, it is particularly preferred that L₂₀ corresponds to formula (XIII), wherein a, b, * and *** are as defined above.

The linker-drug conjugate of the invention offers multiple advantages for the development of new ADCs. It constitutes a modular platform that can easily be adapted to various payloads and/or antibody coupling groups without major changes to the overall structure and synthesis pathway. In particular, the heterocycle Cy acts as central hub, allowing for easy and independent exchange and modification of the three major components: the drug D, the antibody-coupling group Q and the polysarcosine chain X. The linkers of Q, D and X, i.e., L₁, L₂ and L₃, respectively, may also be exchanged independently to introduce new functionalities or modify various properties of the linker-drug conjugate and resulting ADC.

Notably, the chemistry of the central heterocycle and linkers does not particularly restrict the choice of drug/payload D, the antibody-coupling group Q and/or the antibody to be coupled with the linker-drug conjugate, as long as these components are mutually compatible. Once D and Q (and optionally the antibody AB) are chosen, the remaining components may be readily adapted and optimized to tune the release mechanism as well as the physicochemical and pharmacokinetic properties of the resulting ADC, which influence the stability, toxicity and efficacy of the ADC.

For instance, hydrophilicity of the linker-drug conjugate may be tuned by changing the number of sarcosine units in X attached to the central heterocycle Cy. Thus, the linker-drug conjugate may easily be adapted to various payloads with different hydrophobicity, without immediate effect on the spacing or relative configuration of the drug D and the antibody-coupling group Q. When introducing new payloads for ADCs, the system may be used to screen different lengths of the PSAR unit X, thus allowing for straightforward optimization of the compound. Similarly, and independently of X, the linker L₂ may be exchanged in a straightforward manner to test different release mechanisms for the drug D. Thus, the present invention provides a platform that allows for fast and cost-effective screening and optimization of the linker structure for new payloads, thereby streamlining development of new ADCs.

### METHOD FOR PREPARING AN ADC

In a further aspect, the present invention provides a method for preparing an antibody-drug conjugate, comprising the steps of
(i) providing a linker-drug conjugate of the invention as described above, and
(ii) reacting the linker-drug conjugate with a functional group of an antibody or antigen-binding fragment thereof.

The linker-drug conjugate in step (i) may be provided by chemical synthesis procedures as described in Examples 1-3, using chemical reactions and conditions generally known by one skilled in the art.

For example, the polysarcosine unit X may be synthesized with solid-phase peptide synthesis (SPPS) using standard Fmoc chemistry. Depending on the heterocycle Cy, Cy may be linked to the PSAR unit during SPPS, such as if Cy is a proline derivative as described in Example 3 (LP4). Alternatively, Cy may be linked to the PSAR unit in solution, such as if Cy is a piperazine derivative as described in Example 2 (LP2).

In preferred embodiments, L₂ and the drug D may be attached to Cy by attaching an azide-bearing moiety to Cy and performing a copper-catalyzed azide-alkyne cycloaddition with a conjugate of the drug D with a self-immolative spacer bearing an alkyne moiety (see e.g. synthesis of compound 2-6 in Example 2 and compound 4-3 in Example 3). This click chemistry reaction is well-known in the art and yields a triazole, e.g. the triazole of formula (XI).

The linker L₁ may be attached to Cy before or after attaching the azide-bearing moiety to Cy, but preferably before introducing the drug D into the molecule. If the antibody-coupling group Q in E is a maleimide group, E may be introduced by reacting a free amino group of L₁ with 3-maleimidopropionic acid N-hydroxysuccinimide ester to obtain the final linker-drug conjugate. Such a reaction is shown, e.g., in the respective final synthesis steps of Examples 2 and 3.

As described above and shown in the Example section, synthesis of the linker-drug conjugate is straightforward and generally proceeds with good yields. Further, as will be understood by one skilled in the art, the modular nature of the linker-drug conjugate may allow for easy adaptation of parts of the structure without major changes to the remaining synthesis pathway.

Antibodies and antigen-binding fragments thereof are well-known to one skilled in the art. The antibody or antigen-binding fragment thereof used in step (ii) are not particularly limited. Preferably, the antibody recognizes an antigen that is enriched on diseased cells, preferably cancer cells. For example, the antibody may be trastuzumab, which recognizes HER2 as antigen.

The term "antibody" is used in the broadest sense and includes polyclonal and monoclonal antibodies, including intact antibodies and functional (antigen-binding) antibody fragments thereof, including fragment antigen binding (Fab) fragments, F(ab')2 fragments, Fab' fragments, Fv fragments, recombinant IgG (rigG) fragments, single chain antibody fragments, including single chain variable fragments (sFv or scFv), and single domain antibodies (for example, sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, for example, bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD. The antibody can comprise a human IgG1 constant region. The antibody can comprise a human IgG4 constant region.

An "antigen-binding fragment" of an antibody refers to a molecule other than an intact antibody that comprises a portion of an intact antibody that binds the antigen to which the intact antibody binds. Examples of antibody fragments include, but are not limited to, Fv, Fab, Fab', Fab' -SH, F(ab')2; diabodies; linear antibodies; single-chain antibody molecules (for example, scFv or sFv); and multispecific antibodies formed from antibody fragments. In particular embodiments, the antibodies are single-chain antibody fragments comprising a variable heavy chain region and/or a variable light chain region, such as scFvs. Generally, an antibody fragment or antigen-binding fragment will comprise one or more CDRs from a parental antibody that are sufficient to confer binding specificity.

In certain embodiments, the antibody of the invention may be a chimeric antibody, a multispecific antibody, in particular a bispecific antibody, a human antibody, a humanized antibody, or an antigen-binding fragment thereof.

Generally, human or humanized antibodies are preferred. The term "humanized antibody" or "humanized version of an antibody" refers to antibodies for which both heavy and light chains are humanized as a result of antibody engineering. The term "human antibody", as used herein, is intended to include antibodies having variable and constant regions derived from human germ line immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production.

According to the present invention, a "chimeric" antibody refers to an antibody in which a portion of the heavy and/or light chain is derived from a particular source or species, while the remainder of the heavy and/or light chain is derived from a different source or species.

"Multispecific antibodies" bind two or more different epitopes. The epitopes may be on the same or different antigens. A preferred example of a multispecific antibody is a "bispecific antibody" or an antigen-binding fragment thereof which binds two different epitopes. A special subset of bispecific antibodies is a "biparatopic" antibody or antigen-binding fragment, in which each antigen-binding domain recognizes unique, non-overlapping epitopes on the same target antigen.

Further, the antibodies described herein may comprise cysteine insertions for the site-specific coupling of linker payloads. This is particularly useful for maleimide-based linkers, which react specifically with free thiol groups.

The antibody or the antigen-binding fragment thereof may be produced in a suitable host cell comprising a nucleic acid molecule, e.g., a DNA molecule, encoding an antibody VH region, or an antibody VL region, or encoding a complete antibody or an antibody fragment, or a vector or vector system, i.e. a plurality of vectors, comprising said nucleic acid molecule(s), preferably in operative linkage with an expression control sequence, particularly with a heterologous expression control sequence. The host cell may be any known host cell for producing antibodies or antibody fragments, e.g., a prokaryotic cell such as an E. coli cell, a yeast cell, an insect cell, or a mammalian cell, e.g., a CHO cell or a hybridoma cell.

Preferably, step (ii) of the method of the invention comprises a specific reaction of the antibody-coupling group of the linker-drug conjugate, e.g. of Q, with a functional group of the antibody, either a native functional group or one introduced during or after production of the antibody. In a preferred embodiment, step (ii) comprises a maleimide-thiol reaction or a HIPS reaction.

In preferred embodiments, the linker-drug conjugate of the invention comprises a maleimide group as antibody-coupling group Q, and step (ii) of the method of the invention comprises reacting the maleimide with a thiol group in the antibody, preferably with a cysteine residue. The reaction of thiols with maleimides produces a stable thiosuccinimide product and is well-known in the art. Preferably, step (ii) of the method of the invention is conducted in an aqueous buffer at a pH of about 6.5-7.0.

In a further aspect, the present invention relates to the use of a linker-drug conjugate of the present invention for the preparation of an antibody-drug conjugate.

### COMPOUND / LINKER

In a still further aspect, the present invention provides a compound of formula (IV) for use in an antibody-drug conjugate, or a pharmaceutically acceptable salt thereof, 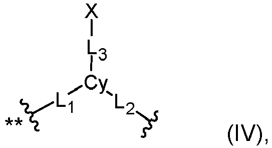 wherein
- Cy: is a 5- or 6-membered saturated, unsaturated or partially unsaturated heterocycle comprising 1-3 nitrogen atoms,
- X: is 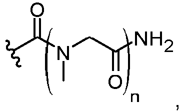
- L₁: is a linker comprising 1-12 consecutive ethylene glycol units and/or a peptide spacer comprising 1-8 amino acids,
- L₂: is 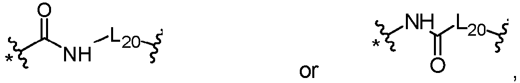
- L₂₀: is a linker comprising one or more units selected from
- one or more C₁-C₁₀-alkylene units,
- a triazole unit having the formula 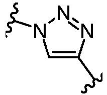
- a peptide comprising 2-4 amino acids, and
- a self-immolative spacer, particularly a self-immolative spacer with an enzyme-cleavable trigger moiety,
- L₃: is a bond or a linker independently defined as L₁, and
- n: is an integer from 1-100, preferably 5-20, more preferably 8-12,
- *: indicates the bond to Cy, and
- **: indicates the antibody-facing end when the compound is part of an antibody-drug conjugate.

When used in an antibody-drug conjugate, the compound as defined above corresponds to the linker which conjugates the antibody to the drug or payload, wherein the drug or payload is preferably linked to L₂ in formula (IV).

Preferably, Cy, L₁, L₂, L₂₀, L₃ and/or n in formula (IV) are as defined above and have preferred embodiments as described above. More preferably, each of Cy, L₁, L₂, L₂₀, L₃ and n in formula (IV) corresponds to a preferred embodiment of Cy, L₁, L₂, L₂₀, L₃ and n, respectively, as described above.

In a particularly preferred embodiment, the compound of the invention corresponds to any one of formulas (V) or (VI), 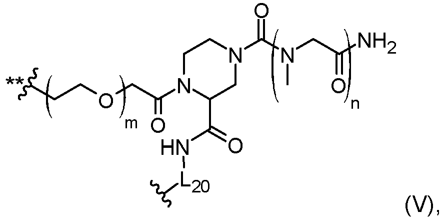 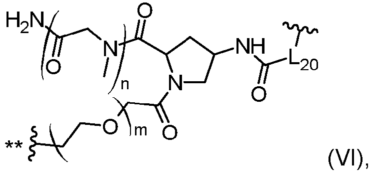 wherein
- L₂₀: is a linker selected from
a C₁-C₁₀-alkylene, 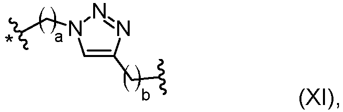 and 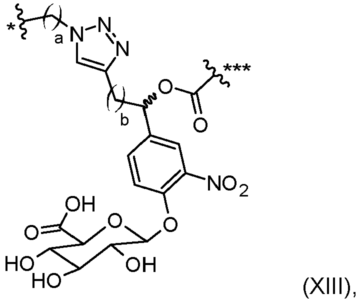
- a: is an integer from 1-8, preferably 1-5, more preferably 1-3,
- b: is an integer from 1-8, preferably 1-5, more preferably 1-2,
- m: is an integer from 1-10, preferably 1-6, more preferably 1-4, and
- n: is an integer from 5-20, preferably 8-12, more preferably 10,
- *: indicates the bond closer to Cy,
- **: indicates the antibody-facing end when the compound is part of an antibody-drug conjugate, and
- ***: indicates the bond to D, preferably the bond to the nitrogen of a primary or secondary amine group in D.

Further preferred embodiments of the various variables are as set forth herein above.

Preferably, the compound of the invention as defined above is a linker in an antibody-drug conjugate.

### ANTIBODY-DRUG CONJUGATE

In a further aspect, the present invention provides an antibody-drug conjugate of formula (VII), or a pharmaceutically acceptable salt thereof, 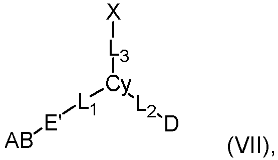 wherein
- Cy: is a 5- or 6-membered, saturated, unsaturated or partially unsaturated heterocycle comprising 1-3 nitrogen atoms,
- AB: is an antibody or antigen-binding fragment thereof,
- D: is a drug or an active agent,
- E': is *-NH-C(=O)-(C₁-C₁₀-alkylene)-Q'-**,
- Q': is an antibody coupling group after the coupling reaction, or a bond,
- X: is 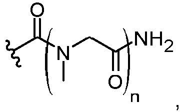
- L₁: is a linker comprising 1-12 consecutive ethylene glycol units and/or a peptide spacer comprising 1-8 amino acids,
- L₂: is 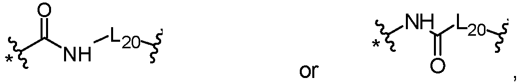
- L₂₀: is a linker comprising one or more units selected from
- one or more C₁-C₁₀-alkylene units,
- a triazole unit having the formula 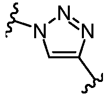
- a peptide comprising 2-4 amino acids, and
- a self-immolative spacer, particularly a self-immolative spacer with an enzyme-cleavable trigger moiety,
- L₃: is a bond or a linker independently defined as L₁,
- n: is an integer from 1-100, preferably 5-20, more preferably 8-12,,
- *: indicates the bond closer to Cy, and
- **: indicates the bond to AB in the formula.

In a preferred embodiment of the ADC of the invention, Cy, D, L₁, L₂, L₂₀, L₃ and/or n in formula (VII) are defined and have preferred embodiments as described above. More preferably, each of Cy, D, L₁, L₂, L₂₀, L₃ and n in formula (VII) corresponds to a preferred embodiment of Cy, D, L₁, L₂, L₂₀, L₃ and n, respectively, as described above.

In the ADC of the invention, the moiety E' comprising Q' is preferably a reaction product of an antibody, preferably of a reactive group in an amino acid residue of an antibody, with the antibody-coupling group comprised in or consisting of moiety E in a linker-drug conjugate of the invention as described above. Thus, E' in the ADC of the invention is preferably *-NH-C(=O)-(C₁-C₅-alkylene)-Q'-**, more preferably *-NH-C(=O)-(C₁-C₃-alkylene)-Q'-**, even more preferably *-NH-C(=O)-(CH₂)₂-Q'-**. Preferably, the alkylene group in E is a saturated linear alkylene group.

Preferably, Q' is an antibody coupling group after the coupling reaction, preferably after the specific coupling reaction to a residue in the antibody. In a preferred embodiment, Q' is a thiosuccinimide product of the reaction of a maleimide group Q with a thiol group in the antibody, preferably represented by the formula 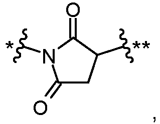 wherein * indicates the bond closer to Cy, and ** indicates the bond to a thiol group of AB in formula (VII). Preferably, Q' is covalently bound to the thiol group of a cysteine residue of AB in formula (VII).

In an alternative embodiment, E' is the product of the reaction of a group E comprising a leaving group Q, which is preferably Br or I, with a thiol group in the antibody. In this case, E' is preferably *-NH-C(=O)-(C₁-C₅-alkylene)-**, more preferably *-NH-C(=O)-(C₁-C₃-alkylene)-**, wherein * indicates the bond closer to Cy, and ** indicates the bond to a thiol group of AB in formula (VII), preferably to the thiol group of cysteine residue of AB. In this case, Q' is the bond between the alkylene in E' and the thiol group of AB.

In a further alternative embodiment, Q' is the product of a HIPS ligation with an aldehyde moiety previously introduced into the antibody as described above.

AB in the antibody-drug conjugate of the present invention is preferably an antibody or antigen-binding fragment thereof as defined above. In particular, the antibody or antigen-binding fragment may be as defined above for antibodies or antigen-binding fragments which may be used in the method for preparing an ADC according to the present invention. Preferably, AB is a monoclonal antibody or antigen-fragment thereof. In a preferred embodiment, AB is trastuzumab.

Preferably, an ADC according to the invention is obtainable by the method for preparing an ADC according to the invention as described above.

In preferred embodiments, the antibody-drug conjugate of the invention corresponds to any one of formulas (VIII) or (IX), 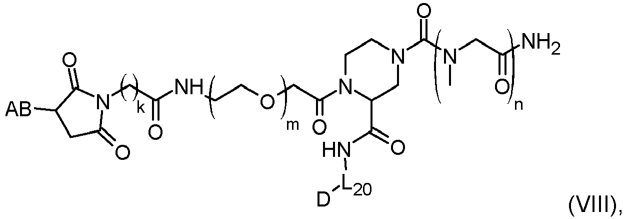 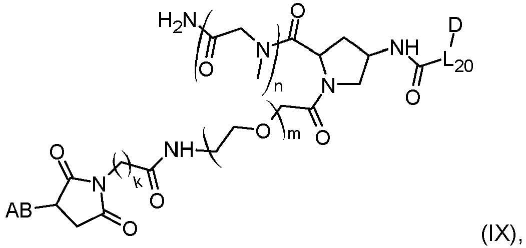 wherein
- AB: is an antibody or antigen-binding fragment thereof,
- D: is a drug or an active agent,
- L₂₀: is a linker selected from
a C₁-C₁₀-alkylene, 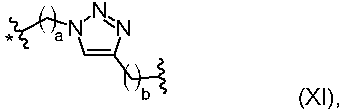 and 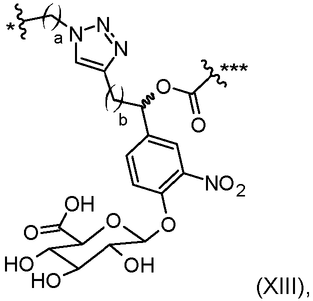
- a: is an integer from 1-8, preferably 1-5, more preferably 1-3,
- b: is an integer from 1-8, preferably 1-5, more preferably 1-2,
- m: is an integer from 1-10, preferably 1-6, more preferably 1-4, and
- n: is an integer from 5-20, preferably 8-12, more preferably 10,
- k: is an integer from 1-8, preferably 1-5, more preferably 1-3,
- *: indicates the bond closer to Cy, and
- ***: indicates the bond to D, preferably the bond to the nitrogen of a primary or secondary amine group in D,
wherein AB is preferably bound to the remaining molecule via a thiol group of a cysteine residue.

Further preferred embodiments of the various variables are as set forth herein above.

In certain embodiments, the antibody-drug conjugate has a molar drug-antibody/antibody fragment ratio (DAR) of greater than 1, i.e., more than one drug molecule is attached to an antibody/antibody fragment. Typically, the conjugate has a DAR of about 2:1 to about 16:1, particularly of about 4:1 to about 10:1 and more particularly of about 6:1 to about 8:1. The DAR may be calculated from a statistical distribution according to known methods. If the DAR is >1, it is preferred that each copy of drug D is conjugated to a different residue in the antibody or antigen-binding fragment thereof, preferably to a different cysteine residue, wherein D is present as part of a structure of formula (XVI), 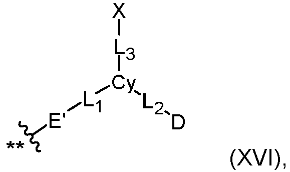 wherein Cy, X, D, E', L₁, L₂ and L₃ are as defined above and ** indicates the bond to the respective residue in the antibody or antigen-binding fragment thereof.

### PHARMACEUTICAL COMPOSITIONS

A further aspect of the present invention is a pharmaceutical composition comprising an antibody-drug conjugate as described herein and a pharmaceutically acceptable carrier. The antibody-drug conjugate is preferably the active agent in the pharmaceutical composition.

The pharmaceutically acceptable carrier may be any suitable carrier or excipient known in the art. Examples of suitable carriers and excipients for formulating antibodies and antibody drug conjugates include saline and aqueous buffer solutions. For example, an ADC of the invention may be provided in a 20 mM histidine/histidine HCl buffer comprising 8% sucrose, preferably having a pH of about 6.0. Typically, the pharmaceutical composition is adapted for parenteral administration, e.g., for subcutaneous, intramuscular, or intravenous injection or by infusion. In further embodiments, the pharmaceutical composition is adapted for local administration, e.g., for intravesical instillation into the bladder.

Depending on the stage and the severity of the disorder, the pharmaceutical composition may be administered once or several times in a therapeutically effective dose to a subject in need thereof, particularly to a human subject. For example, it may be administered once or several times daily, each second day, two times weekly or weekly for a suitable period, e.g., of at least one week, or at least one month. In particular, the attending physician will be able to choose alternative administration regimens based on the medical needs of the patient.

### MEDICAL APPLICATIONS

According to a further aspect of the invention, the antibody-drug conjugate or pharmaceutical composition as described above is used in medicine, including human and veterinary medicine, particularly in human medicine. Thus, in one aspect, the present invention relates to an antibody-drug conjugate or a pharmaceutical composition as described above for use in medicine, including human and veterinary medicine, particularly in human medicine.

In particular, the antibody-drug conjugate or pharmaceutical composition of the invention may be used in the treatment of cancer, particularly of cancer in a human patient.

In therapeutic applications, the active agent is administered in an effective amount to a subject in need thereof, particularly to a human subject. The dose will depend on the specific type of agent, e.g., type of antibody or antibody fragment, the type of disease, and the mode of administration, e.g., locally or systemically.

In the prevention and/or treatment of cancer, a therapeutic dose of an antibody or antibody drug conjugate is typically from about 0.3 mg/kg to about 10 mg/kg.

The antibody-drug conjugate may be administered alone or together with a further active agent, which may be selected from chemotherapeutic agents, e.g., anti-metabolites, alkylating agents, intercalating agents, or anti-mitotic agents), inhibitors of specific kinases e.g., tyrosine kinase inhibitors, serine/threonine kinase inhibitors or phosphoinositide kinase inhibitors, immunotherapeutic compounds, e.g., immune checkpoint inhibitors, CAR-T cells, bi- or multispecific immune cell engager such as NK- or T-cell engagers, or therapeutic vaccines or oncolytic viruses.

The following examples should further illustrate the described embodiments without limiting the scope of the invention.

### EXAMPLES

### Example 1: Synthetic Procedure for Exatecan conjugated with a self-immolative spacer bearing an alkyne group (compound 3)

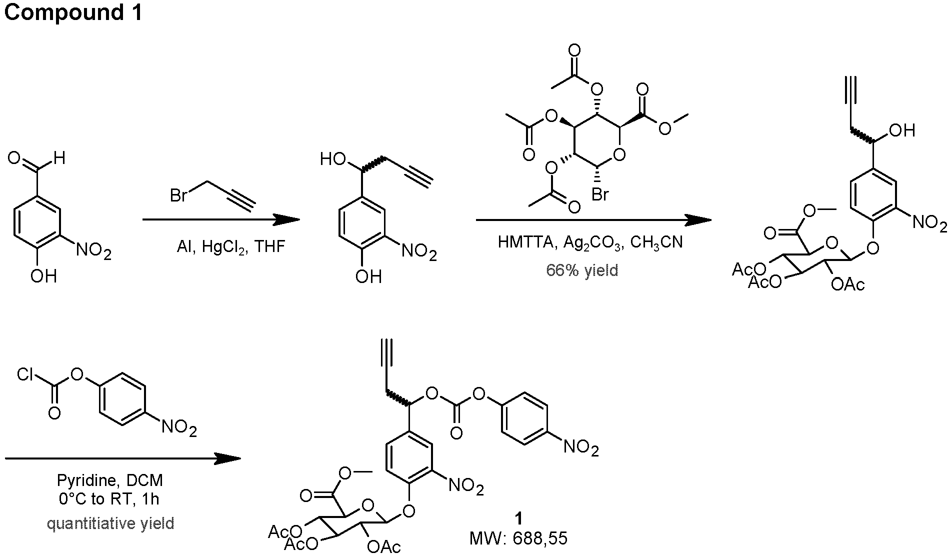

Propargyl bromide (80%, in solution, 1 eq.) in toluene was added dropwise into anhydrous THF, containing aluminum (1 eq.) and HgCl₂ (catalytic amount). After 6 hrs of reflux, the reaction mixture was cooled down to 0°C, and 4-hydroxy-3-nitrobenzaldehyde (0.2 eq.) in THF was added dropwise. The reaction mixture was stirred for about 30 mins, hydrolyzed with HCl 1N (10 mL) and extracted with ethyl acetate. After silica gel chromatography, a dark brown oil was isolated.

It was then added (1 eq.), together with 1-bromo-(2,3,4,6-O-tetra-O-acetyl)-D-galactopyranoside (2 eq.), at 0°C, to a solution of HMTTA (0.7 eq.) and Ag₂CO₃ (3.7 eq.) in anhydrous acetonitrile previously stirred at 25°C for 2 hrs. The reaction mixture was stirred for 4 hrs at 25°C. The reaction was then quenched with water and extracted with ethyl acetate. After silica gel chromatography, a colorless solid was obtained.

The obtained compound (1 eq.) was then stirred in dichloromethane and pyridine (2.5 eq.). para-nitrophenyl chloroformate (2 eq.) was added at 0°C and the reaction mixture was stirred for 1 hr, hydrolyzed with HCl 1N and extracted with dichloromethane. After silica gel chromatography, a colorless solid was obtained.

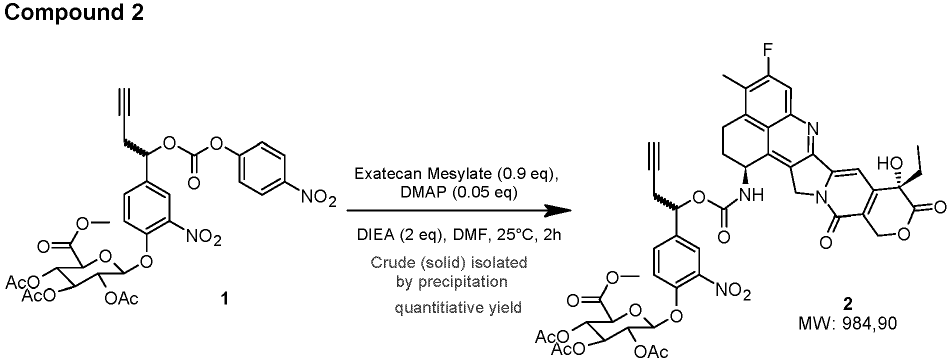

To a solution of compound 1 (1 eq.) and exatecan (0.9 eq.) in DMF was added DMAP (0.05 eq.) and DIEA (2 eq.). The mixture was stirred at 25°C for 2 hrs. Acid was added to the solution until pH<7, then the compound was precipitated by isopropyl ether. Compound 2 (crude) was isolated as a yellow solid (93.9% purity by HPLC). LCMS: Rt = 0.56 min, MS cal.: 984.9, MS observed: [M+H]⁺ = 985.4.

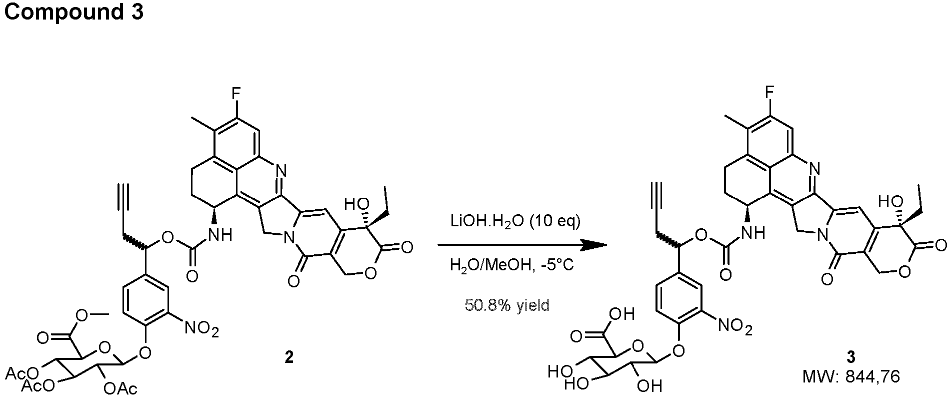

To a solution of compound 2 (1 eq.) in H₂O (10.0 mL) and MeOH (40.0 mL) was added LiOH.H₂O (10.0 eq.). The mixture was stirred at -5°C for 5 mins. Acid was added to the solution until pH<7. The solution was purified by prep-HPLC (TFA condition). Compound 3 (50.8% yield) was obtained as a yellow solid (47.9%: 50.9% purity by HPLC, mixture of the two enantiomers). LCMS: Rt = 0.48 min, MS cal.: 844.7, MS observed: [M+H]⁺ = 845.3.

### Example 2: Synthetic Procedure for linker-drug conjugate LP2

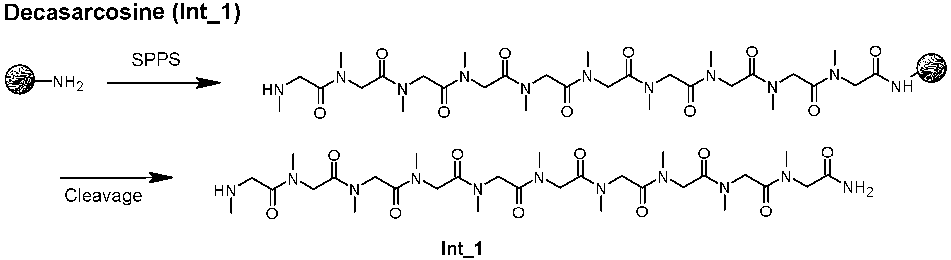

### Peptide Synthesis:

The peptide was synthesized using solid-phase peptide synthesis (SPPS).
1) Resin preparation: Add DMF to the vessel containing Sieber Amide Resin (1 eq.) and swell for 2 hrs.
2) Deprotection: 20% piperidine in DMF was added and the resin was agitated under N₂ at 25°C for 30 mins. The resin was washed with DMF (x 5) and filtered to get the resin.
3) Coupling: A solution of Fmoc-SAR-OH and DIEA in DMF was added to the resin, then HATU/HBTU was added. The mixture was agitated with N₂ at 20°C for 30 mins. The resin was washed with DMF (x 4).
4) Repeat above step 2 to 3 for the coupling of following SAR units (2-10):

| **#** | **Coupling compound** | **Coupling reagents** |
|---|---|---|
| 1 | Fmoc-SAR-OH (2 eq.) | HBTU (1.9 eq.) and DIEA (4 eq.) |
| 2 | Fmoc-SAR-OH (2 eq.) | HBTU (1.9 eq.) and DIEA (4 eq.) |
| 3 | Fmoc-SAR-OH (2 eq.) | HATU (1.9 eq) and DIEA (4 eq.) |
| 4 | Fmoc-SAR-OH (2 eq.) | HATU (1.9 eq) and DIEA (4 eq.) |
| 5 | Fmoc-SAR-OH (2 eq.) | HATU (1.9 eq) and DIEA (4 eq.) |
| 6 | Fmoc-SAR-OH (3 eq.) | HATU (2.85 eq) and DIEA (6 eq.) |
| 7 | Fmoc-SAR-OH (3 eq.) | HATU (2.85 eq) and DIEA (6 eq.) |
| 8 | Fmoc-SAR-OH (3 eq.) | HATU (2.85 eq) and DIEA (6 eq.) |
| 9 | Fmoc-SAR-OH (3 eq.) | HATU (2.85 eq) and DIEA (6 eq.) |
| 10 | Fmoc-SAR-OH (3 eq.) | HATU (2.85 eq) and DIEA (6 eq.) |

### Peptide Cleavage and Purification:

1) Add cleavage solution (% TFA/DCM) to the flask containing resin at 20°C, stir for 3 hrs and filter.
2) The filtrate was concentrated to afford the crude product.
3) The crude peptide was purified by prep-HPLC and pure Int_1 was obtained as a white solid.

### Purification conditions:

| **Separation condition** | |
|---|---|
| Dissolution condition | Dissolve in 30% MeCN/H₂O |
| Instrument | DAC-150 |
| Mobile Phase | A: H₂O (0.1% TFA in H₂O) |
| | B: CH₃CN |
| Gradient | 0-30-30min, Retention time: 12.5 min |
| Column | Welch Ultimate^{®} XB-C18, 250*50 mm, 7 um, 120Å |
| Flow Rate | 80 mL/min |
| Wavelength | 220/254 nm |
| Oven Tem. | Room temperature |

### Peptide 1

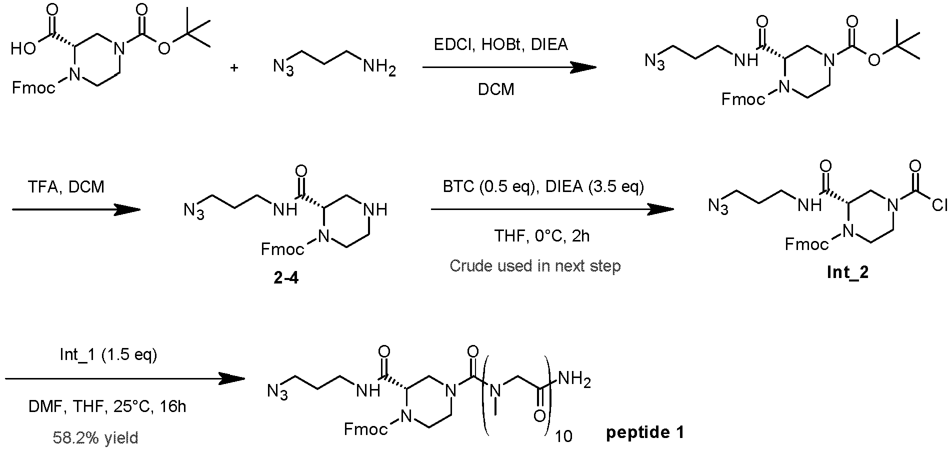

Fmoc protected piperazine (1 eq.) and 2-azidoethanamine (1 eq.) are mixed in dichloromethane with EDCI (1 eq.) and HOBt (1.2 eq.) and DIEA (3 eq.). After quenching, extraction and concentration, the compound (crude) is stirred in DCM and TFA (50%) for Boc deprotection. After quenching and extraction, a solution of compound 2-4 (1 eq.) in THF and DIEA (3.50 eq.) was added dropwise at 0°C. BTC (0.5 eq.) in THF was then added dropwise at 0°C. The mixture was stirred at 0°C for 2 hrs. **Int_1** (1.5 eq.) in DMF and THF (1:1, v:v) was then added dropwise at 0°C. The resulting mixture was stirred at 25°C for 16 hrs. The residue was purified by HPLC. **Peptide 1** (58.2% yield, 91.6% purity by HPLC) was obtained as a pink solid. LCMS: Rt = 0.872 min, Exact mass: 1188.3, MS observed: [M+H] ⁺ = 1189.5.

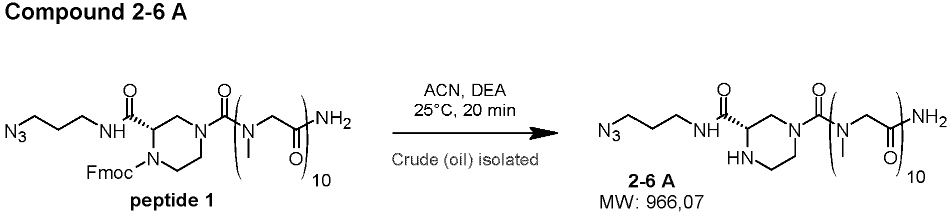

To a solution of **peptide 1** (1 eq.) in MeCN was added DEA (30%). The mixture was stirred at 25°C for 20 mins. The reaction mixture was concentrated under reduced pressure to provide compound **2-6 A** (crude, 95.6% purity by HPLC) as a white oil. LCMS: Rt = 0.24 min, MS cal.: 966.0, MS observed: [M+H]⁺ = 966.5.

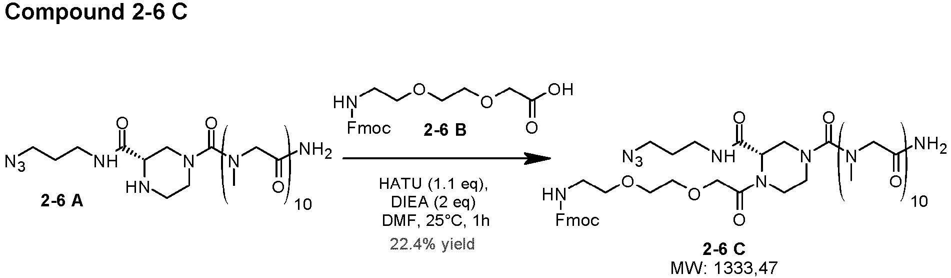

DIEA (2 eq) was added to a solution of compound **2-6 B** (1 eq.) and HATU (1.1 eq.) in DMF. The mixture was stirred for 5 mins at 25°C followed by the addition of **2-6 A** (1 eq.). The mixture was stirred at 25°C for 1 hr. The reaction mixture was filtered to give a solution. The solution was purified by prep-HPLC. Compound **2-6 C** (22.4% yield, 97.9% purity by HPLC) was obtained as a white solid. LCMS: Rt = 0.44 min, MS cal.: 1333.4, MS observed: [M+H]⁺ = 1333.9.

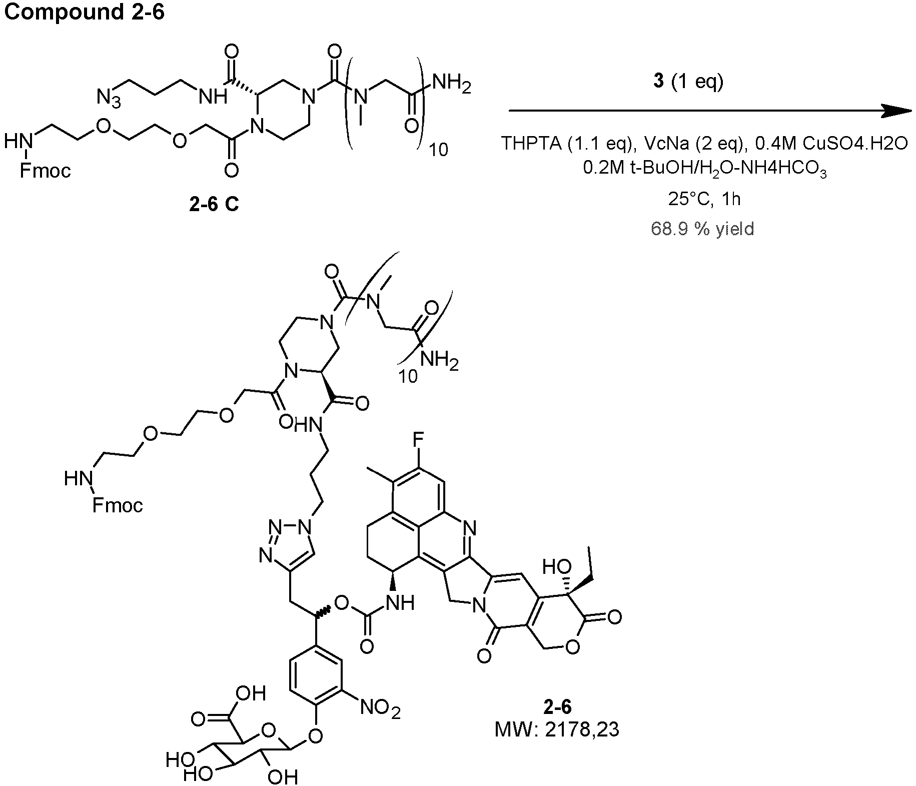

A mixture of compound **2-6 C** (1 eq.), compound **3** (1 eq.), THPTA (1.1 eq.), VcNa (2 eq.) and CuSO₄.5H₂O (1.10 eq.) in 0.2 M *^{t}*BuOH/H₂O-NH₄HCO₃ was degassed, and purged with N₂ for 3 times. The mixture was stirred at 25°C for 1 hr under N₂ atmosphere. The solution was purified by prep-HPLC. Compound **2-6** (68.9% yield, 93.0% purity by HPLC) was obtained as a yellow solid. LCMS: Rt = 0.47 min, MS cal.: 2178.2, MS observed: [M/2+H]⁺ = 1090.1.

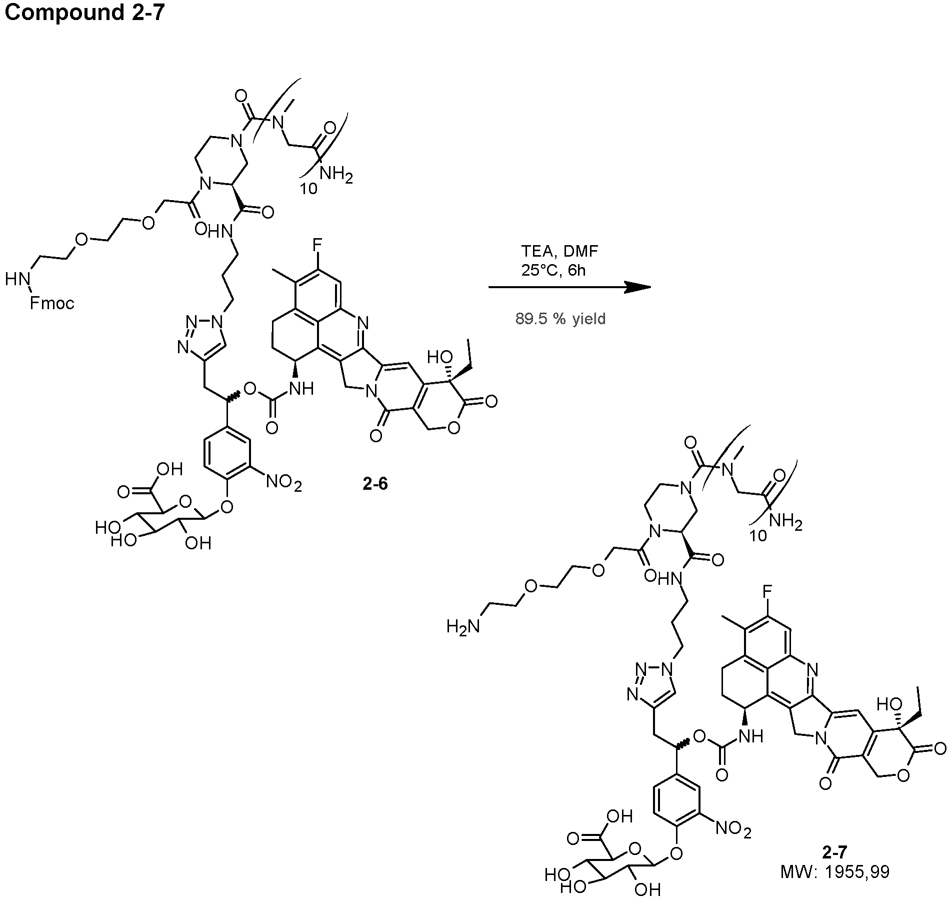

To a solution of compound **2-6** (1 eq.) in DMF was added TEA (20%). The mixture was stirred at 25°C for 6 hrs. The solution was purified by prep-HPLC. Compound **2-7** (89.5% yield, 57.0% purity by HPLC) was obtained and isolated as a yellow solid. LCMS: Rt = 0.36 min, MS cal.: 1955.9, MS observed: [M/2+H]⁺ = 979.0.

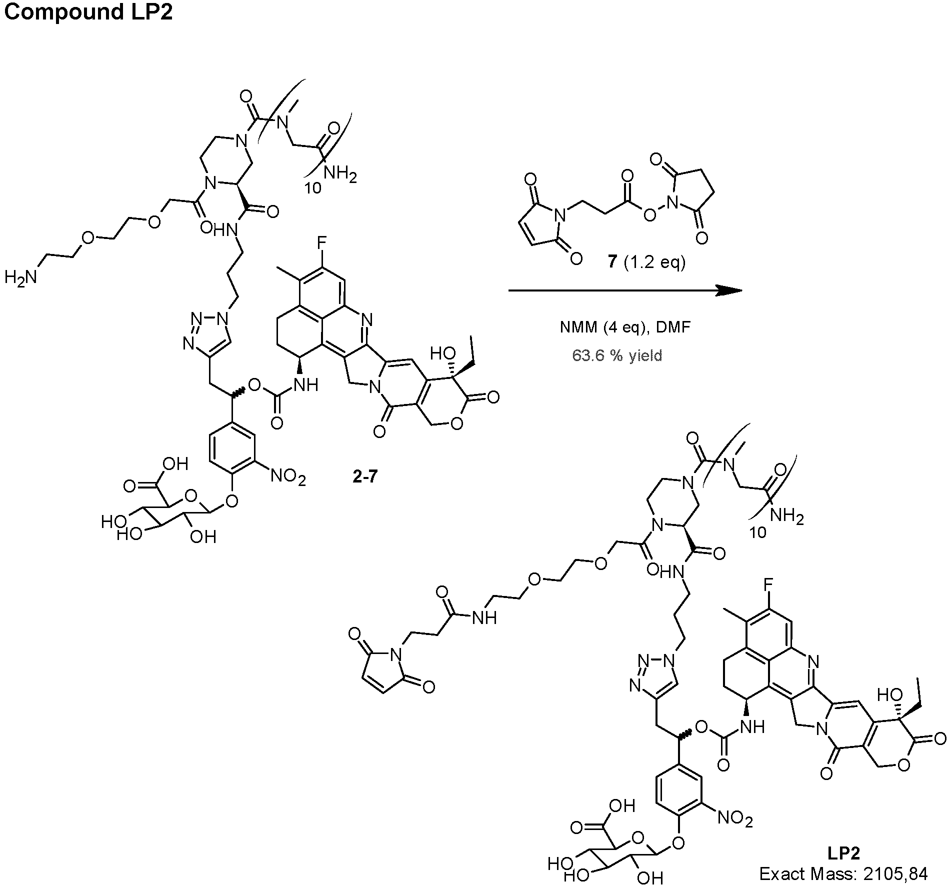

NMM (4 eq.) was added to a solution of compound **2-7** (1 eq.) and compound **7** (1.2 eq.) in DMF. The mixture was stirred at 25°C for 2 hrs. The solution was purified by prep-HPLC. **LP2** (63.6% yield, 97.3% purity by HPLC) was obtained and isolated as a yellow solid. LCMS: Rt = 0.40 min, MS cal.: 2107.1, MS observed: [M/2+H]⁺ = 1054.5.

### Example 3: Synthetic Procedure for linker-drug conjugate LP4

### Peptide 3

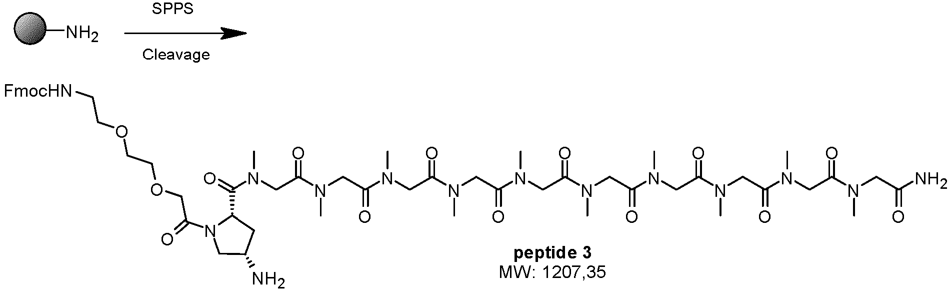

### Peptide Synthesis:

The peptide was synthesized using standard SPPS.
1) Resin preparation: Added DMF to the vessel containing Sieber Amide Resin (1 eq.) and swell for 2 hrs.
2) Deprotection: 20% piperidine in DMF was added, agitated the resin with N₂ at 25 °C for 3 mins. The resin was washed with DMF (x 5) and filtered to get the resin.
3) Coupling: A solution of Fmoc-SAR-OH (2 eq.) (coupling compound) and DIEA (4 eq.) in DMF was added to the resin followed by HATU (1.9 eq.). The mixture was agitated with N₂ at 20°C for 30 mins. The resin was washed with DMF (x 4).
4) Repeated above step 2 to 3 for the coupling of following amino acids (2-12):

| **#** | **Coupling compound** | **Coupling reagents** |
|---|---|---|
| 1 | Fmoc-SAR-OH (2 eq.) | HATU (1.9 eq.) and DIEA (4 eq.) |
| 2 | Fmoc-SAR-OH (2 eq.) | HATU (1.9 eq.) and DIEA (4 eq.) |
| 3 | Fmoc-SAR-OH (2 eq.) | HATU (1.9 eq.) and DIEA (4 eq.) |
| 4 | Fmoc-SAR-OH (2 eq.) | HATU (1.9 eq.) and DIEA (4 eq.) |
| 5 | Fmoc-SAR-OH (2 eq.) | HATU (1.9 eq.) and DIEA (4 eq.) |
| 6 | Fmoc-SAR-OH (3 eq.) | HATU (2.85 eq.) and DIEA (6 eq.) |
| 7 | Fmoc-SAR-OH (3 eq.) | HATU (2.85 eq.) and DIEA (6 eq.) |
| 8 | Fmoc-SAR-OH (3 eq.) | HATU (2.85 eq.) and DIEA (6 eq.) |
| 9 | Fmoc-SAR-OH (3 eq.) | HATU (2.85 eq.) and DIEA (6 eq.) |
| 10 | Fmoc-SAR-OH (3 eq.) | HATU (2.85 eq.) and DIEA (6 eq.) |
| 11 | Boc-cis-4-N-Fmoc-Pro-OH (2 eq.) | HATU (1.9 eq.) and DIEA (4 eq.) |
| 12 | Fmoc-PEG2-CH2COOH | HATU (1.9 eq.) and DIEA (4 eq.) |

### Peptide Cleavage and Purification:

1) Added cleavage solution (50% TFA/DCM) to the flask containing resin at 20°C, stirred for 2.5 hrs and filtered.
2) The filtrate was concentrated to afford the crude product.
3) The crude peptide was purified by prep-HPLC and **peptide 3** (97.4% purity by HPLC) was isolated as a white solid.

### Purification conditions:

| **Separation condition** | |
|---|---|
| Dissolution condition | Dissolve in 30% MeCN/H₂O |
| Instrument | DAC-150 |
| Mobile Phase | A: H₂O (0.1% TFA in H₂O) |
| | B: CH₃CN |
| Gradient | 14-44-30min, Retention time: 21 min |
| Column | Welch Ultimate^{®} XB-C18, 250*50 mm, 7 um, 120Å |
| Flow Rate | 80 mL/min |
| Wavelength | 220/254 nm |
| Oven Tem. | Room temperature |

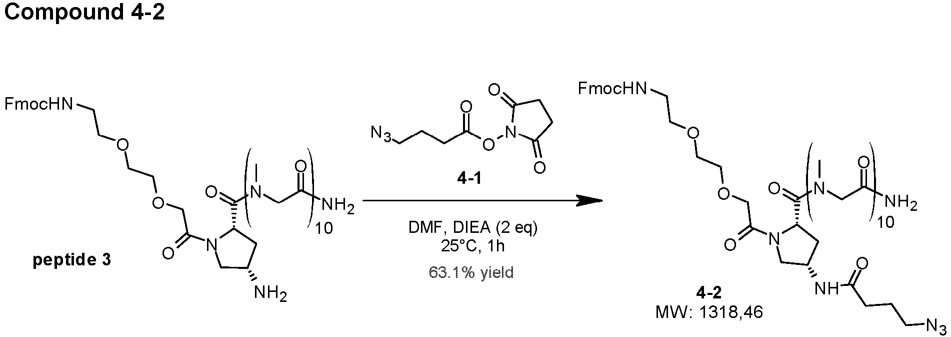

To a solution of Peptide 3 (1 eq.) and compound 4-1 (1 eq.) in DMF were added DIEA (2 eq.). The mixture was stirred at 25°C for 1 hr. The solution was purified by prep-HPLC and Compound 4-2 (63.1% yield, 96.7% purity by HPLC) was obtained and isolated as a white solid. LCMS: Rt = 0.44 min, MS cal.: 1318.5, MS observed: [M+H]⁺ = 1318.9.

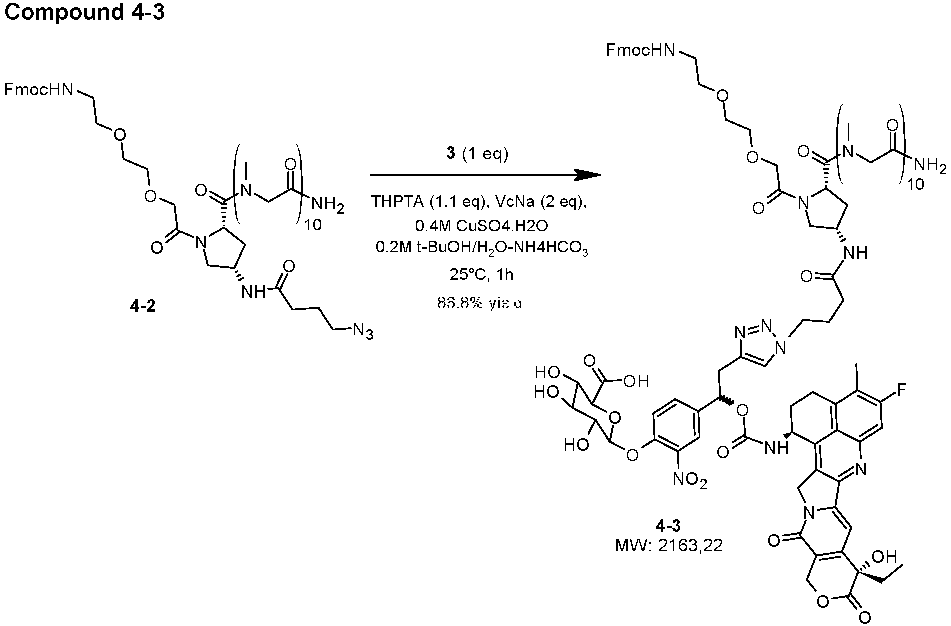

A mixture of compound 4-2 (1 eq.), compound 3 (1 eq.), THPTA (1.1 eq.), CuSO₄.5H₂O (1.10 eq.) and VcNa (2 eq.) in 0.2 M t-BuOH/H₂O-NH₄HCO₃ were degassed and purged with N₂ 3 times. The mixture was stirred at 25°C for 1 hr under N₂ atmosphere. The solution was purified by prep-HPLC. Compound 4-3 (86.8% yield, 92.3% purity by HPLC) was obtained and isolated as a yellow solid. LCMS: Rt = 0.47 min, MS cal.: 2163.2, MS observed: [M/2+H]⁺ = 1082.6.

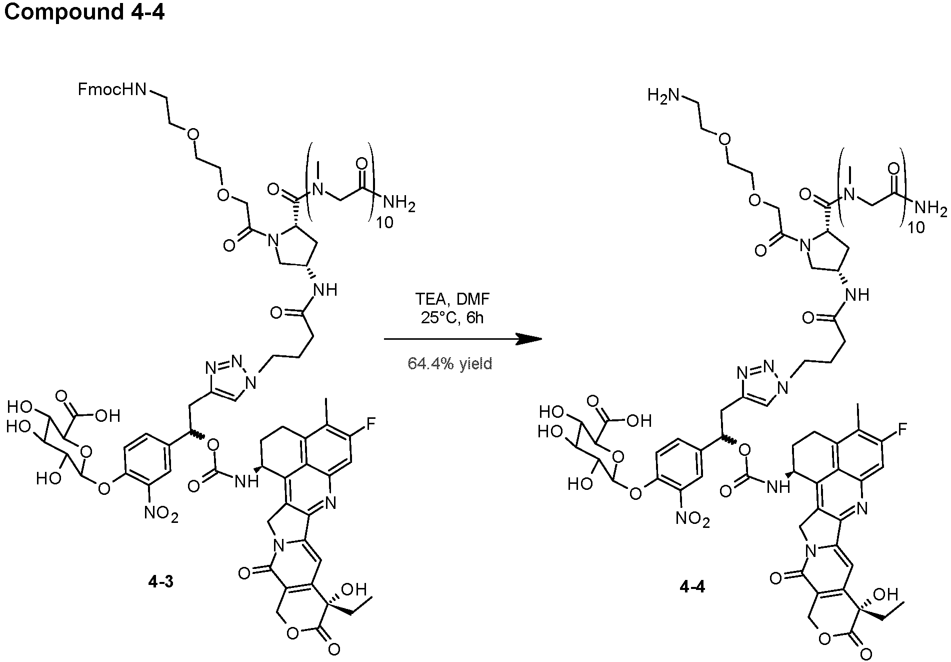

To a solution of compound **4-3** (1 eq.) in DMF was added TEA (20%). The mixture was stirred at 25°C for 6 hrs. The solution was purified by prep-HPLC. Compound **4-4** (64.4% yield, 96.9% purity by HPLC) was obtained as a yellow solid. LCMS: Rt = 0.36 min, MS cal.: 1940.9, MS observed: [M+H]⁺ = 1941.1.

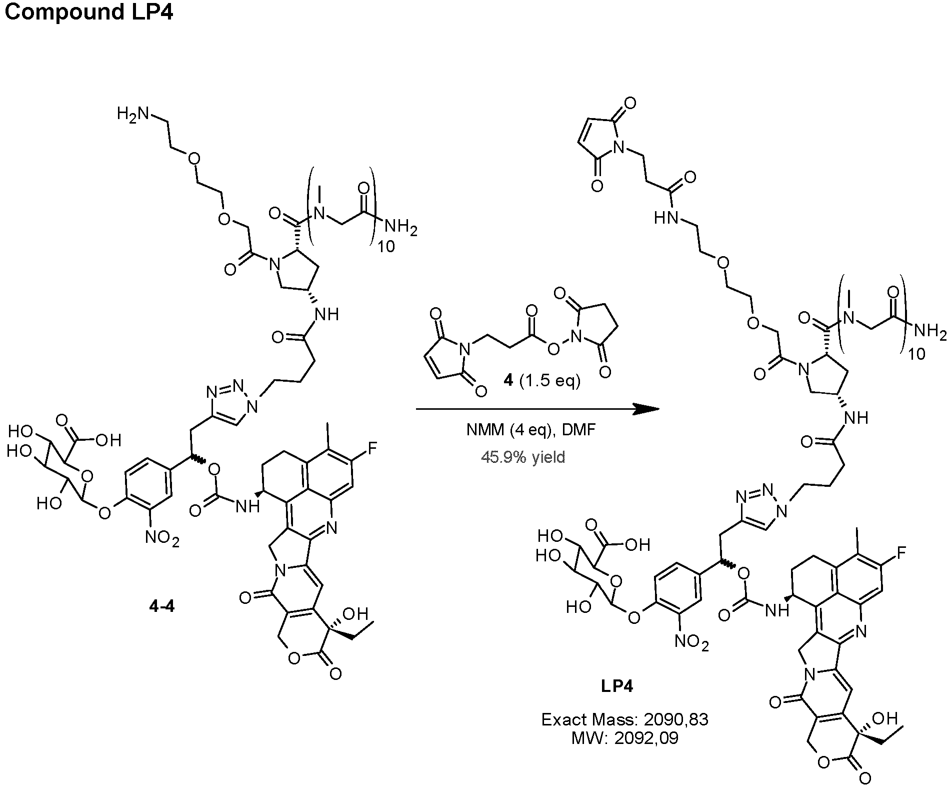

To a solution of compound **4-4** (1 eq.) and compound **4** (1.5 eq.) in DMF were added NMM (4 eq.). The mixture was stirred at 25°C for 2 hrs. The solution was purified by prep-HPLC. **LP4** (45.9% yield, 96.9% purity by HPLC) was obtained and isolated as a yellow solid. LCMS: Rt = 0.40 min, MS cal.: 2092.1, MS observed: [M/2+H]⁺ = 1047.0.

The present invention is summarized by the following items:

### [Linker-drug conjugate]

1. A linker-drug conjugate of formula (I), or a pharmaceutically acceptable salt thereof, 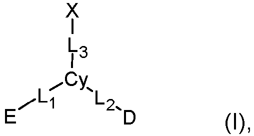 wherein
   - Cy: is a 5- or 6-membered saturated, unsaturated or partially unsaturated heterocycle comprising 1-3 nitrogen atoms,
   - D: is a drug or an active agent,
   - E: is -NH-C(=O)-(C₁-C₁₀-alkylene)-Q,
   - Q: is an antibody coupling group or a leaving group,
   - X: is 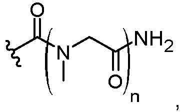
   - L₁: is a linker comprising 1-12 consecutive ethylene glycol units and/or a peptide spacer comprising 1-8 amino acids,
   - L₂: is 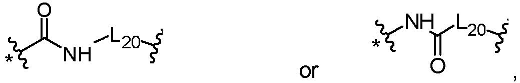
   - L₂₀: is a linker comprising one or more units selected from
   - one or more C₁-C₁₀-alkylene units,
   - a triazole unit having the formula 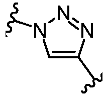
   - a peptide comprising 2-4 amino acids, and
   - a self-immolative spacer, particularly a self-immolative spacer with an enzyme-cleavable trigger moiety,
   - L₃: is a bond or a linker independently defined as L₁, and
   - n: is an integer from 1-100, preferably 5-20, more preferably 8-12,
   wherein * indicates the bond to Cy.
2. The linker-drug conjugate according to item 1, wherein all heteroatoms in Cy are nitrogen atoms.
3. The linker-drug conjugate according to item 1 or 2, wherein Cy is a fully saturated heterocycle.
4. The linker-drug conjugate according to item 1 or 2, wherein Cy is an aromatic heterocycle.
5. The linker-drug conjugate according to any one of the preceding items, wherein Cy is selected from the group consisting of a pyrrolidine unit, a piperazine unit, an imidazole unit, a pyrazole unit, a 1,2,4-triazole unit and a 1,2,3-triazole unit, wherein Cy is preferably a pyrrolidine unit or a piperazine unit.
6. The linker-drug conjugate according to any one of the preceding items, wherein 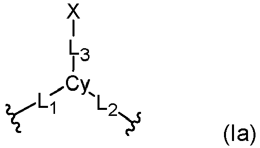 is 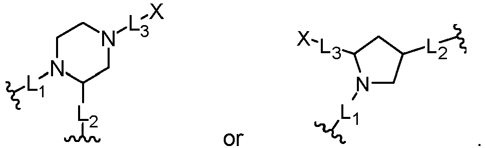
7. The linker-drug conjugate according to any one of the preceding items, wherein n is an integer from 8 to 12, more preferably 10.
8. The linker-drug conjugate according to any one of the preceding items, wherein L₁ is covalently bound to a nitrogen atom in Cy, preferably via a peptide bond.
9. The linker-drug conjugate according to any one of the preceding items, wherein L₁ is 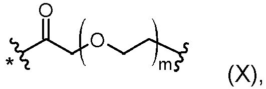
   wherein m is an integer from 1-12, preferably 1-10, more preferably 1-6, even more preferably 1-4, particularly 2, and
   wherein * indicates the bond to Cy, preferably to a nitrogen atom in Cy.
10. The linker-drug conjugate according to any one of items 1-8, wherein L₁ comprises a peptide spacer comprising 1-8 amino acids, preferably 1-4 amino acids.
11. The linker-drug conjugate according to any one of items 1-8 and 10, wherein L₁ comprises 1-12 consecutive ethylene glycol units, preferably 1-4 ethylene glycol units, and a peptide spacer comprising 1-8 amino acids, preferably 1-4 amino acids.
12. The linker-drug conjugate according to any one of the preceding items, wherein L₂ is covalently bound to a carbon atom in Cy.
13. The linker-drug conjugate according to any one of the preceding items, wherein L₂₀ is a C₁-C₁₀-alkylene, preferably a C₁-C₅-alkylene, more preferably a C₁-C₃-alkylene.
14. The linker-drug conjugate according to any one of items 1-12, wherein L₂₀ comprises or is 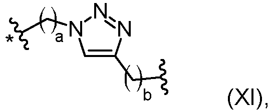 wherein
   - a: is an integer from 1-8, preferably 1-5, more preferably 1-3, in particular 3,
   - b: is an integer from 1-8, preferably 1-5, more preferably 1-2, in particular 1, and
   - *: indicates the bond closer to Cy.
15. The linker-drug conjugate according to any one of items 1-12 or 14, wherein L₂₀ comprises or is a peptide comprising 2-4 amino acids, wherein the N terminus of the peptide is preferably closer to Cy.
16. The linker-drug conjugate according to item 15, wherein the peptide is sensitive to cleavage by a lysosomal peptidase, particularly by cathepsin, preferably wherein the cleavage site is at the C terminus of the peptide.
17. The linker-drug conjugate according to item 15 or 16, wherein the peptide is selected from valine-citrulline (Val-Cit), Val-Ala, cyclobutane-1,1-dicarboxamide (cBu)-Cit and GGFG, preferably wherein the peptide is Val-Cit or GGFG.
18. The linker-drug conjugate according to any one of items 1-12 or 14-17, wherein L₂₀ comprises a self-immolative spacer, preferably a self-immolative spacer comprising a para-N- or *O*-substituted benzyl carbamate unit such as a para-aminobenzyl carbamate (PABC) unit, optionally comprising a nitro group in *meta* position.
19. The linker-drug conjugate according to item 18, wherein the self-immolative spacer is covalently bound to D, preferably to the nitrogen of a primary or secondary amine group in D.
20. The linker-drug conjugate according to any one of items 1-12 or 14-19, wherein L₂₀ comprises a self-immolative spacer with an enzyme-cleavable trigger moiety, wherein the enzyme-cleavable trigger moiety is preferably a sugar or a sulfate group, more preferably β-D-glucuronic acid, β-D-galactose or a sulfate group.
21. The linker-drug conjugate according to any one of items 1-12 or 14-20, wherein L₂₀ comprises a self-immolative spacer corresponding to formula (XII): 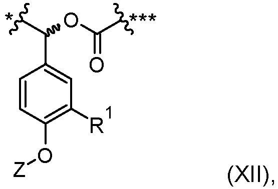 wherein
   - Z: is an enzyme-cleavable trigger moiety preferably selected from 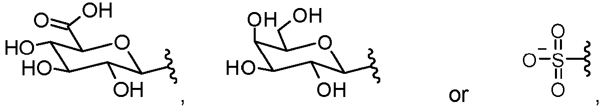 preferably wherein Z is 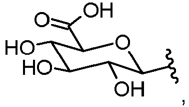
   - R¹: is NO₂ or H, preferably NO₂,
   - *: indicates the bond closer to Cy, and
   - ***: indicates the bond closer to D, preferably a bond to D, more preferably a bond to the nitrogen of a primary or secondary amine group in D.
22. The linker-drug conjugate according to item 21, wherein L₂₀ comprises and preferably corresponds to a structure of formula (XIII): 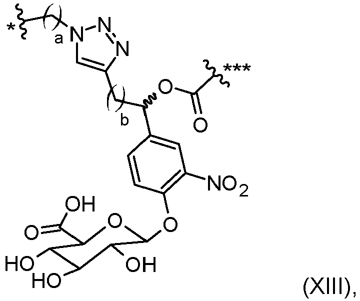 wherein
   - a: is an integer from 1-8, preferably 1-5, more preferably 1-3, in particular 3,
   - b: is an integer from 1-8, preferably 1-5, more preferably 1-2, in particular 1,
   - *: indicates the bond closer to Cy, and
   - ***: indicates a bond to D, preferably a bond to the nitrogen of a primary or secondary amine group in D.
23. The linker-drug conjugate according to any one of items 1-12 or 14-19, wherein L₂₀ comprises a structure according to formula (XIV): 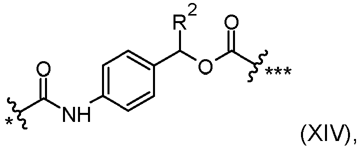 wherein
   - R²: is H, R²⁰ or C(=O)R²⁰, preferably H or C(=O)R²⁰, more preferably H,
   - R²⁰: is selected from C₁-C₂₄ (hetero)alkyl groups, C₃-C₁₀ (hetero)cycloalkyl groups, C₂-C₁₀ (hetero)aryl groups, C₃-C₁₀ alkyl(hetero)aryl groups and C₃-C₁₀ (hetero)arylalkyl groups, which are optionally substituted and optionally interrupted by one or more heteroatoms selected from O, S and NR²¹ with R²¹ being independently selected from H or C₁-C₄ alkyl groups, preferably 4-methyl-piperazine or morpholine,
   - *: indicates the bond closer to Cy, and
   - ***: indicates the bond closer to D, preferably a bond to D, more preferably a bond to the nitrogen of a primary or secondary amine group in D.
24. The linker-drug conjugate according to any one of items 1-12, 14-19 or 23, wherein L₂₀ comprises a peptide according to any one of items 15-17 and a self-immolative spacer according to any one of items 18-19 and 23.
25. The linker-drug conjugate according to item 24, wherein the peptide is a dipeptide selected from Val-Cit, Val-Ala, and cBu-Cit and the self-immolative spacer is a PABC spacer as defined in item 23, wherein the peptidic carbonyl in formula (XIV) is the C-terminal carbonyl of the dipeptide.
26. The linker drug conjugate according to any one of items 1-12 or 14-20, wherein L₂₀ comprises a structure of formula (XV): 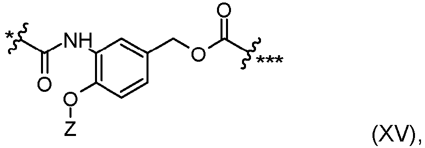 wherein Z is an enzyme-cleavable trigger moiety and is preferably 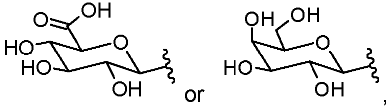 more preferably 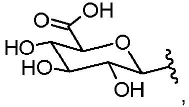 and wherein * indicates the bond closer to Cy, and *** indicates the bond closer to D, preferably a bond to D, more preferably a bond to the nitrogen of a primary or secondary amine group in D.
27. The linker-drug conjugate according to any one of the preceding items, wherein L₃ is covalently bound to a nitrogen atom or a carbon atom in Cy.
28. The linker-drug conjugate according to any one of the preceding items, wherein L₃ is a covalent bond between X and Cy, preferably between X and a nitrogen atom or a carbon atom in Cy.
29. The linker-drug conjugate according to any one of items 1-27, wherein L₃ is a linker independently defined as L₁, preferably wherein L₃ is a linker as defined in any one of items 8-12 for L₁.
30. The linker-drug conjugate according to any one of the preceding items, wherein E is - NH-C(=O)-(C₁-C₅-alkylene)-Q, preferably -NH-C(=O)-(C₁-C₃-alkylene)-Q, more preferably -NH-C(=O)-(CH₂)₂-Q.
31. The linker-drug conjugate according to any one of the preceding items, wherein Q is a thiol-reactive group, preferably a maleimide group.
32. The linker-drug conjugate according to any one of items 1-30, wherein Q is a leaving group, preferably selected from Br and I.
33. The linker-drug conjugate according to any one of the preceding items, wherein D is a drug or active agent bearing a reactive amine, preferably exatecan.
34. The linker-drug conjugate according to any one of the preceding items, corresponding to any one of formulas (II) or (III), 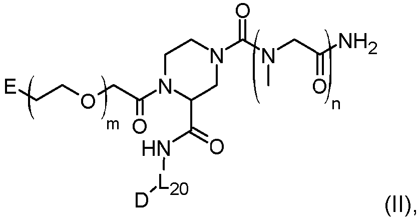 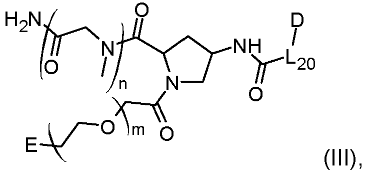 wherein
   - D: is a drug or an active agent,
   - E: is -NH-C(=O)-(C₁-C₃-alkylene)-Q,
   - Q: is an antibody coupling group or a leaving group, preferably a maleimide group,
   - L₂₀: is a linker selected from
   a C₁-C₁₀-alkylene, preferably C₁-C₅-alkylene, more preferably C₁-C₃-alkylene, 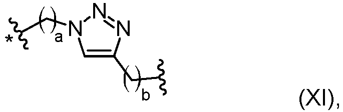 and 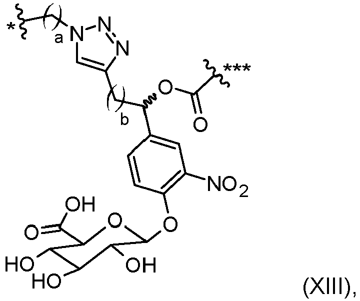
   - a: is an integer from 1-8, preferably 1-5, more preferably 1-3,
   - b: is an integer from 1-8, preferably 1-5, more preferably 1-2,
   - m: is an integer from 1-10, preferably 1-6, more preferably 1-4, and
   - n: is an integer from 5-20, preferably 8-12, more preferably 10,
   - *: indicates the bond closer to Cy, and
   - ***: indicates the bond to D, preferably the bond to the nitrogen of a primary, secondary or tertiary amine group in D.

### [Method]

35. A method for preparing an antibody-drug conjugate, comprising the steps of
(i) providing a linker-drug conjugate according to any one of the preceding items, and
(ii) reacting the linker-drug conjugate with a functional group of an antibody or antigen-binding fragment thereof.

36. The method of item 35, wherein the linker-drug conjugate comprises a maleimide group as antibody-coupling group Q, and step (ii) comprises reacting the maleimide with a thiol group in the antibody, preferably with a cysteine residue.

### [Use]

37. Use of a linker-drug conjugate according to any one of items 1-34 for the preparation of an antibody-drug conjugate.

### [Compound/ linker]

38. A compound of formula (IV) for use in an antibody-drug conjugate, or a pharmaceutically acceptable salt thereof, 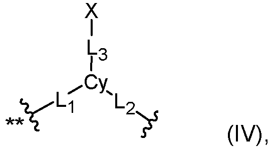 wherein
- Cy: is a 5- or 6-membered saturated, unsaturated or partially unsaturated heterocycle comprising 1-3 nitrogen atoms,
- X: is 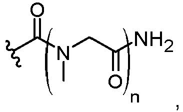
- L₁: is a linker comprising 1-12 consecutive ethylene glycol units and/or a peptide spacer comprising 1-8 amino acids,
- L₂: is 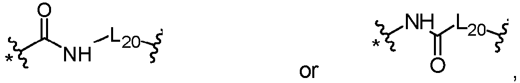
- L₂₀: is a linker comprising one or more units selected from
- one or more C₁-C₁₀-alkylene units,
- a triazole unit having the formula 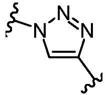
- a peptide comprising 2-4 amino acids, and
- a self-immolative spacer, particularly a self-immolative spacer with an enzyme-cleavable trigger moiety,
- L₃: is a bond or a linker independently defined as L₁, and
- n: is an integer from 1-100, preferably 5-20, more preferably 8-12,
- *: indicates the bond to Cy, and
- **: indicates the antibody-facing end when the compound is part of an antibody-drug conjugate.

39. The compound of item 38, wherein Cy, L₁, L₂, L₂₀, L₃ and/or n are defined as in any one of items 1-34.

40. The compound according to any one of items 38-39, corresponding to any one of formulas (V) or (VI), 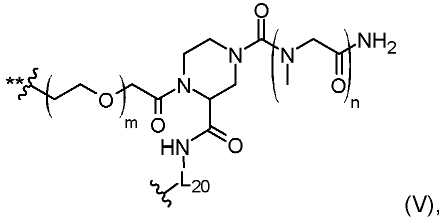 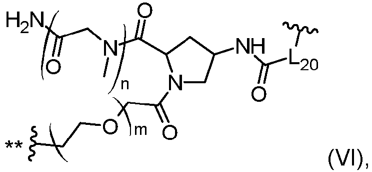 wherein
- L₂₀: is a linker selected from
a C₁-C₁₀-alkylene, 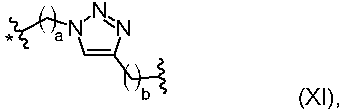 and 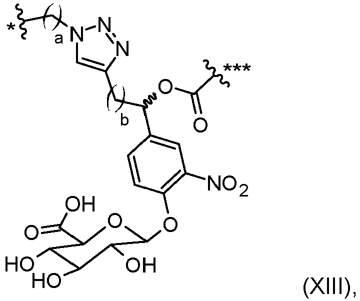
- a: is an integer from 1-8, preferably 1-5, more preferably 1-3,
- b: is an integer from 1-8, preferably 1-5, more preferably 1-2,
- m: is an integer from 1-10, preferably 1-6, more preferably 1-4, and
- n: is an integer from 5-20, preferably 8-12, more preferably 10,
- *: indicates the bond closer to Cy,
- **: indicates the antibody-facing end when the compound is part of an antibody-drug conjugate, and
- ***: indicates the bond to D, preferably the bond to the nitrogen of a primary or secondary amine group in D.

### [ADC]

41. An antibody-drug conjugate of formula (VII), or a pharmaceutically acceptable salt thereof, 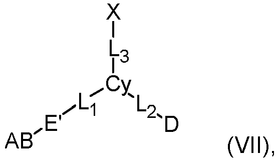 wherein
   - Cy: is a 5- or 6-membered, saturated, unsaturated or partially unsaturated heterocycle comprising 1-3 nitrogen atoms,
   - AB: is an antibody or antigen-binding fragment thereof,
   - D: is a drug or an active agent,
   - E': is *-NH-C(=O)-(C₁-C₁₀-alkylene)-Q'-**,
   - Q': is an antibody coupling group after the coupling reaction, or a bond,
   - X: is 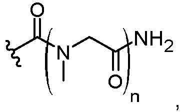
   - L₁: is a linker comprising 1-12 consecutive ethylene glycol units and/or a peptide spacer comprising 1-8 amino acids,
   - L₂: is 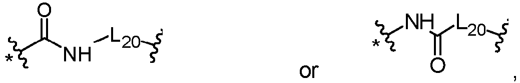
   - L₂₀: is a linker comprising one or more units selected from
   - one or more C₁-C₁₀-alkylene units,
   - a triazole unit having the formula 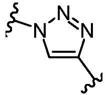
   - a peptide comprising 2-4 amino acids, and
   - a self-immolative spacer, particularly a self-immolative spacer with an enzyme-cleavable trigger moiety,
   - L₃: is a bond or a linker independently defined as L₁,
   - n: is an integer from 1-100, preferably 5-20, more preferably 8-12,
   - *: indicates the bond closer to Cy, and
   - **: indicates the bond to AB in the formula.
42. The antibody-drug conjugate according to item 41, wherein Cy, D, L₁, L₂, L₂₀, L₃ and/or n are defined as in any one of items 1-34.
43. The antibody-drug conjugate according to any one of items 41-42, wherein E' is *-NH-C(=O)-(C₁-C₅-alkylene)-Q'-**, preferably *-NH-C(=O)-(C₁-C₃-alkylene)-Q'-**, more preferably *-NH-C(=O)-(CH₂)₂-Q'-**.
44. The antibody-drug conjugate according to any one of items 41-43, wherein Q' is 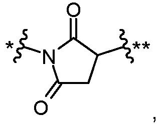 wherein * indicates the bond closer to Cy, and ** indicates the bond to a thiol group of AB in formula (VII).
45. The antibody-drug conjugate according to any one of items 41-43, wherein Q' is a covalent bond.
46. The antibody-drug conjugate according to any of items 41-45, wherein Q' forms a covalent bond to the thiol group of a cysteine residue of AB in formula (VII).
47. The antibody-drug conjugate according to any one of items 41-46, wherein AB is a monoclonal antibody or antigen-binding fragment thereof, preferably trastuzumab.
48. The antibody-drug conjugate according to any one of items 41-47 corresponding to any one of formulas (VIII) or (IX), 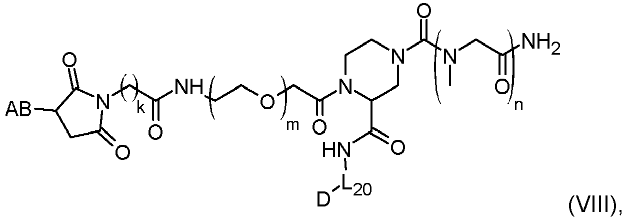 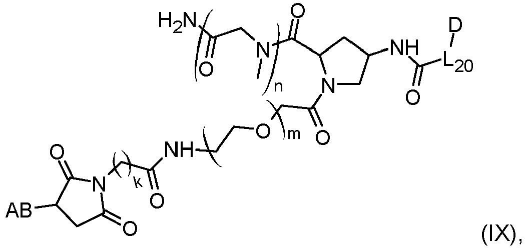 wherein
   - AB: is an antibody or antigen-binding fragment thereof,
   - D: is a drug or an active agent,
   - L₂₀: is a linker selected from
   a C₁-C₁₀-alkylene, 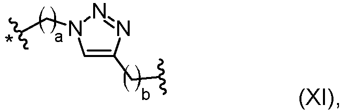 and 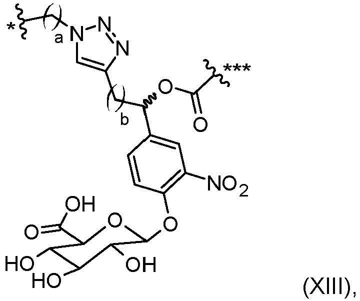
   - a: is an integer from 1-8, preferably 1-5, more preferably 1-3,
   - b: is an integer from 1-8, preferably 1-5, more preferably 1-2,
   - m: is an integer from 1-10, preferably 1-6, more preferably 1-4, and
   - n: is an integer from 1-100, preferably 8-12, more preferably 10,
   - k: is an integer from 1-8, preferably 1-5, more preferably 1-3,
   - *: indicates the bond closer to Cy, and
   - ***: indicates the bond to D, preferably the bond to the nitrogen of a primary, secondary or tertiary amine group in D,
   wherein AB is preferably bound to the remaining molecule via a thiol group of a cysteine residue.
49. A pharmaceutical composition comprising an antibody-drug conjugate according to any one of items 41-48 and a pharmaceutically acceptable carrier.
50. An antibody-drug conjugate according to any one of items 41-48 or a pharmaceutical composition according to item 49 for use in medicine, particularly in human medicine.

## Claims

1. A linker-drug conjugate of formula (I), or a pharmaceutically acceptable salt thereof, 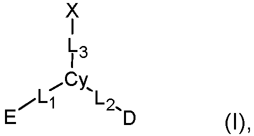 wherein
Cy is a 5- or 6-membered saturated, unsaturated or partially unsaturated heterocycle comprising 1-3 nitrogen atoms,
D is a drug or an active agent,
E is -NH-C(=O)-(C₁-C₁₀-alkylene)-Q,
Q is an antibody coupling group or a leaving group,
X is 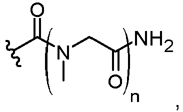
L₁ is a linker comprising 1-12 consecutive ethylene glycol units and/or a peptide spacer comprising 1-8 amino acids,
L₂ is 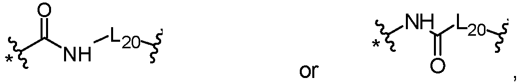
L₂₀ is a linker comprising one or more units selected from
- one or more C₁-C₁₀-alkylene units,
- a triazole unit having the formula 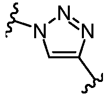
- a peptide comprising 2-4 amino acids, and
- a self-immolative spacer, particularly a self-immolative spacer with an enzyme-cleavable trigger moiety,
L₃ is a bond or a linker independently defined as L₁, and
n is an integer from 1-100, preferably 5-20, more preferably 8-12,
wherein * indicates the bond to Cy.

2. The linker-drug conjugate according to claim 1, wherein all heteroatoms in Cy are nitrogen atoms, and/or wherein Cy is a fully saturated heterocycle or an aromatic heterocycle, particularly a fully saturated heterocycle, preferably wherein Cy is selected from the group consisting of a pyrrolidine unit, a piperazine unit, an imidazole unit, a pyrazole unit, a 1,2,4-triazole unit and a 1,2,3-triazole unit, more preferably wherein Cy is a pyrrolidine unit or a piperazine unit,
and/or
wherein
- L₁ is covalently bound to a nitrogen atom in Cy, preferably via a peptide bond, and/or
- L₂ is covalently bound to a carbon atom in Cy, and/or
- L₃ is covalently bound to a nitrogen atom or a carbon atom in Cy,
preferably wherein 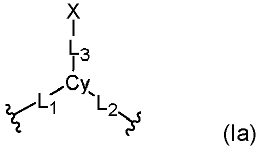 is

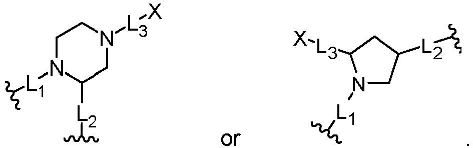

3. The linker-drug conjugate according to any one of the preceding claims,
wherein L₁ comprises a peptide spacer comprising 1-8 amino acids, preferably 1-4 amino acids, and/or
wherein L₁ comprises 1-12 consecutive ethylene glycol units, preferably 1-10, more preferably 1-2, even more preferably 2 consecutive ethylene glycol units, particularly wherein L₁ is 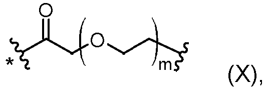
wherein m is an integer from 1 -12, preferably 1-10, more preferably 1-6, even more preferably 1-4, particularly 2, and
wherein * indicates the bond to Cy, preferably to a nitrogen atom in Cy.

4. The linker-drug conjugate according to any one of the preceding claims,
wherein L₂₀ is a C₁-C₁₀-alkylene, preferably a C₁-C₅-alkylene, more preferably a C₁-C₃-alkylene,
or
wherein L₂₀ comprises or is 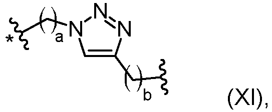 wherein
a is an integer from 1-8, preferably 1-5, more preferably 1-3, in particular 3,
b is an integer from 1-8, preferably 1-5, more preferably 1-2, in particular 1, and
* indicates the bond closer to Cy,
and/or
wherein L₂₀ comprises or is a peptide comprising 2-4 amino acids, wherein the N terminus of the peptide is particularly closer to Cy,
preferably wherein the peptide is sensitive to cleavage by a lysosomal peptidase, particularly by cathepsin, particularly wherein the cleavage site is at the C terminus of the peptide,
more preferably wherein the peptide is selected from valine-citrulline (Val-Cit), Val-Ala, cyclobutane-1,1-dicarboxamide (cBu)-Cit and GGFG, even more preferably wherein the peptide is Val-Cit or GGFG,
and/or
wherein L₂₀ comprises a self-immolative spacer, preferably a self-immolative spacer comprising a para-N- or O-substituted benzyl carbamate unit such as a *para-*aminobenzyl carbamate (PABC) unit, optionally comprising a nitro group in *meta* position,
wherein the self-immolative spacer is preferably covalently bound to D, more preferably to the nitrogen of a primary or secondary amine group in D,
wherein the self-immolative spacer preferably comprises an enzyme-cleavable trigger moiety, wherein the enzyme-cleavable trigger moiety is preferably a sugar or a sulfate group, more preferably β-D-glucuronic acid, β-D-galactose or a sulfate group.

5. The linker-drug conjugate according to any one of the preceding claims, wherein L₂₀ comprises a self-immolative spacer corresponding to formula (XII): 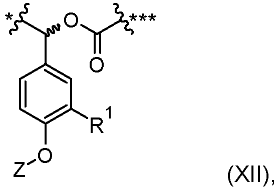 wherein
Z is an enzyme-cleavable trigger moiety preferably selected from 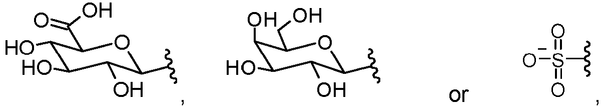 preferably wherein Z is 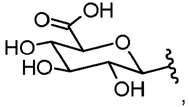
R¹ is NO₂ or H, preferably NO₂,
* indicates the bond closer to Cy, and
*** indicates the bond closer to D, preferably a bond to D, more preferably a bond to the nitrogen of a primary or secondary amine group in D,
preferably wherein L₂₀ comprises and more preferably corresponds to a structure of formula (XIII): 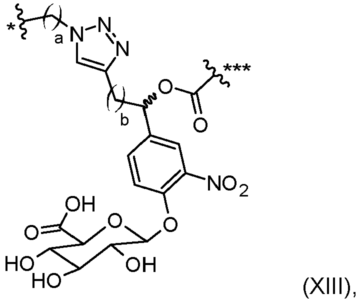 wherein
a is an integer from 1-8, preferably 1-5, more preferably 1-3, in particular 3,
b is an integer from 1-8, preferably 1-5, more preferably 1-2, in particular 1,
* indicates the bond closer to Cy, and
*** indicates a bond to D, preferably a bond to the nitrogen of a primary or secondary amine group in D,
or
wherein L₂₀ comprises a structure of formula (XIV): 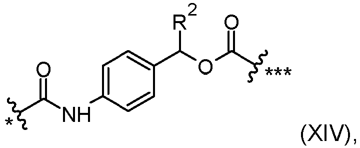 wherein
R² is H, R²⁰ or C(=O)R²⁰, preferably H or C(=O)R²⁰, more preferably H,
R₂₀ is selected from C₁-C₂₄ (hetero)alkyl groups, C₃-C₁₀ (hetero)cycloalkyl groups, C₂-C₁₀ (hetero)aryl groups, C₃-C₁₀ alkyl(hetero)aryl groups and C₃-C₁₀ (hetero)arylalkyl groups, which are optionally substituted and optionally interrupted by one or more heteroatoms selected from O, S and NR²¹ with R²¹ being independently selected from H or C₁-C₄ alkyl groups,
preferably 4-methyl-piperazine or morpholine,
* indicates the bond closer to Cy, and
*** indicates the bond closer to D, preferably a bond to D, more preferably a bond to the nitrogen of a primary or secondary amine group in D,
preferably wherein L₂₀ additionally comprises a dipeptide selected from Val-Cit, Val-Ala, and cBu-Cit, wherein the peptidic carbonyl in formula (XIV) is the C-terminal carbonyl of the dipeptide,
or
wherein L₂₀ comprises a structure of formula (XV): 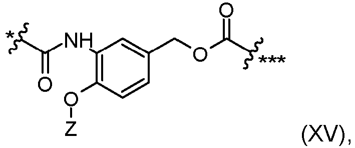
wherein Z is an enzyme-cleavable trigger moiety and is preferably 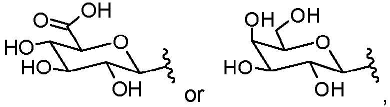
more preferably 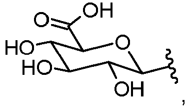
and wherein * indicates the bond closer to Cy, and *** indicates the bond closer to D, preferably a bond to D, more preferably a bond to the nitrogen of a primary or secondary amine group in D.

6. The linker-drug conjugate according to any one of the preceding claims,
wherein L₃ is a covalent bond between X and Cy or a linker independently defined as L₁, wherein the covalent bond is preferably between X and a nitrogen atom or a carbon atom in Cy and the linker is preferably as defined in claim 3 for L₁,
and/or
wherein E is -NH-C(=O)-(C₁-C₅-alkylene)-Q, preferably -NH-C(=O)-(C₁-C₃-alkylene)-Q, more preferably -NH-C(=O)-(CH₂)₂-Q,
and/or
wherein Q is a thiol-reactive group, preferably a maleimide group, or a leaving group, preferably selected from Br and I,
and/or
wherein D is a drug or active agent bearing a reactive amine, preferably exatecan.

7. The linker-drug conjugate according to any one of the preceding claims, corresponding to any one of formulas (II) or (III), 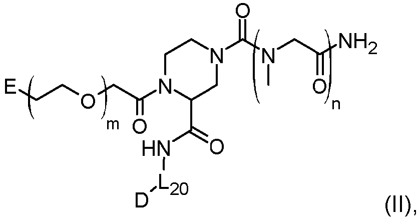 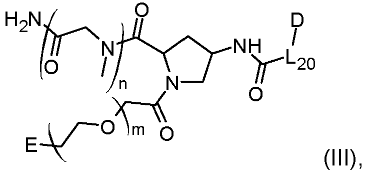 wherein
D is a drug or an active agent,
E is -NH-C(=O)-(C₁-C₃-alkylene)-Q,
Q is an antibody coupling group or a leaving group, preferably a maleimide group,
L₂₀ is a linker selected from
a C₁-C₁₀-alkylene, preferably C₁-C₅-alkylene, more preferably C₁-C₃-alkylene, 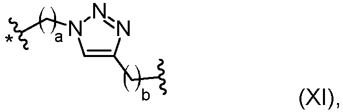 and 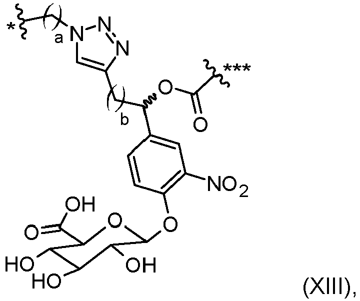
a is an integer from 1-8, preferably 1-5, more preferably 1-3,
b is an integer from 1-8, preferably 1-5, more preferably 1-2,
m is an integer from 1-10, preferably 1-6, more preferably 1-4, and
n is an integer from 5-20, preferably 8-12, more preferably 10,
* indicates the bond closer to Cy, and
*** indicates the bond to D, preferably the bond to the nitrogen of a primary, secondary or tertiary amine group in D.

8. A method for preparing an antibody-drug conjugate, comprising the steps of
(i) providing a linker-drug conjugate according to any one of the preceding claims, and
(ii) reacting the linker-drug conjugate with a functional group of an antibody or antigen-binding fragment thereof,
preferably wherein the linker-drug conjugate comprises a maleimide group as antibody-coupling group Q, and step (ii) comprises reacting the maleimide with a thiol group in the antibody, preferably with a cysteine residue.

9. Use of a linker-drug conjugate according to any one of claims 1-7 for the preparation of an antibody-drug conjugate.

10. A compound of formula (IV) for use in an antibody-drug conjugate, or a pharmaceutically acceptable salt thereof, 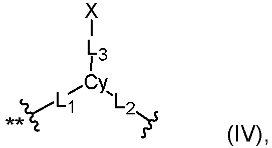 wherein
Cy is a 5- or 6-membered saturated, unsaturated or partially unsaturated heterocycle comprising 1-3 nitrogen atoms,
X is 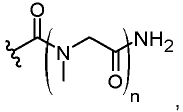
L₁ is a linker comprising 1-12 consecutive ethylene glycol units and/or a peptide spacer comprising 1-8 amino acids,
L₂ is 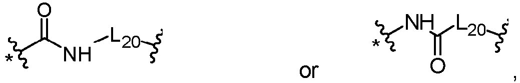
L₂₀ is a linker comprising one or more units selected from
- one or more C₁-C₁₀-alkylene units,
- a triazole unit having the formula 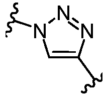
- a peptide comprising 2-4 amino acids, and a self-immolative spacer, particularly a self-immolative spacer with an enzyme-cleavable trigger moiety,
L₃ is a bond or a linker independently defined as L₁, and
n is an integer from 1-100, preferably 5-20, more preferably 8-12,
* indicates the bond to Cy, and
** indicates the antibody-facing end when the compound is part of an antibody-drug conjugate.

11. The compound of claim 10, wherein Cy, L₁, L₂, L₂₀, L₃ and/or n are defined as in any one of claims 1-7, preferably wherein
the compound corresponds to any one of formulas (V) or (VI), 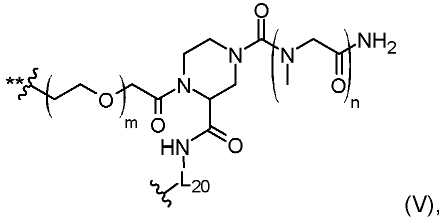 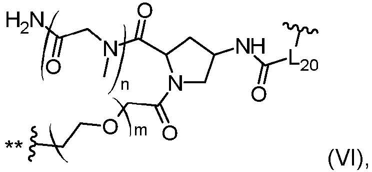 wherein
L₂₀ is a linker selected from
a C₁-C₁₀-alkylene, 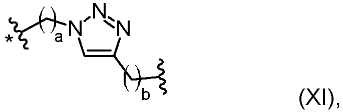 and 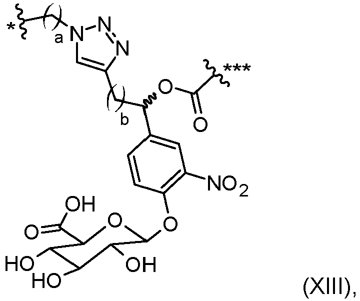
a is an integer from 1-8, preferably 1-5, more preferably 1-3,
b is an integer from 1-8, preferably 1-5, more preferably 1-2,
m is an integer from 1-10, preferably 1-6, more preferably 1-4, and
n is an integer from 5-20, preferably 8-12, more preferably 10,
* indicates the bond closer to Cy,
** indicates the antibody-facing end when the compound is part of an antibody-drug conjugate, and
*** indicates the bond to D, preferably the bond to the nitrogen of a primary or secondary amine group in D.

12. An antibody-drug conjugate of formula (VII), or a pharmaceutically acceptable salt thereof, 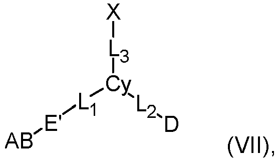 wherein
Cy is a 5- or 6-membered, saturated, unsaturated or partially unsaturated heterocycle comprising 1-3 nitrogen atoms,
AB is an antibody or antigen-binding fragment thereof,
D is a drug or an active agent,
E' is *-NH-C(=O)-(C₁-C₁₀-alkylene)-Q'-**,
Q' is an antibody coupling group after the coupling reaction, or a bond,
X is 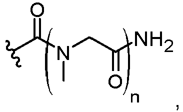
L₁ is a linker comprising 1-12 consecutive ethylene glycol units and/or a peptide spacer comprising 1-8 amino acids,
L₂ is 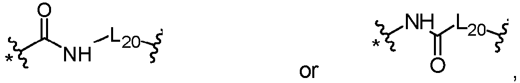
L₂₀ is a linker comprising one or more units selected from
- one or more C₁-C₁₀-alkylene units,
- a triazole unit having the formula 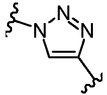
- a peptide comprising 2-4 amino acids, and
- a self-immolative spacer, particularly a self-immolative spacer with an enzyme-cleavable trigger moiety,
L₃ is a bond or a linker independently defined as L₁,
n is an integer from 1-100, preferably 5-20, more preferably 8-12,
* indicates the bond closer to Cy, and
** indicates the bond to AB in the formula.

13. The antibody-drug conjugate according to claim 12, wherein Cy, D, L₁, L₂, L₂₀, L₃ and/or n are defined as in any one of claims 1-7,
and/or
wherein E' is *-NH-C(=O)-(C₁-C₅-alkylene)-Q'-**, preferably *-NH-C(=O)-(C₁-C₃-alkylene)-Q'-**, more preferably *-NH-C(=O)-(CH₂)₂-Q'-**,
and/or
wherein Q' is a covalent bond or is 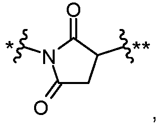
wherein * indicates the bond closer to Cy, and ** indicates the bond to a thiol group of AB in formula (VII), wherein Q' preferably forms a covalent bond to the thiol group of a cysteine residue of AB in formula (VII),
and/or
wherein AB is a monoclonal antibody or antigen-binding fragment thereof, preferably trastuzumab,
preferably wherein the antibody-drug conjugate corresponds to any one of formulas (VIII) or (IX), 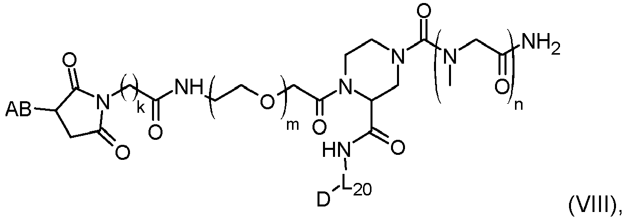 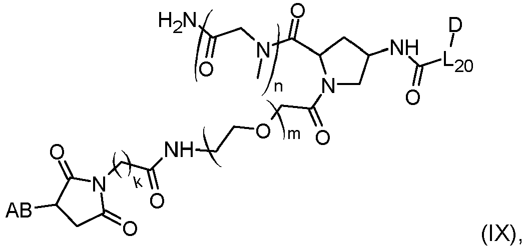
wherein
AB is an antibody or antigen-binding fragment thereof,
D is a drug or an active agent,
L₂₀ is a linker selected from
a C₁-C₁₀-alkylene, 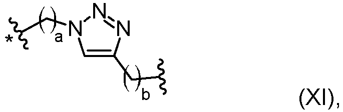 and 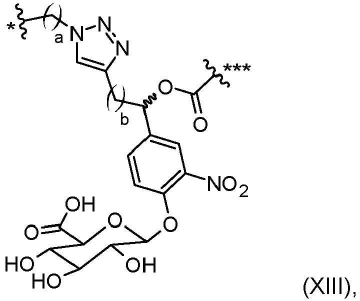
a is an integer from 1-8, preferably 1-5, more preferably 1-3,
b is an integer from 1-8, preferably 1-5, more preferably 1-2,
m is an integer from 1-10, preferably 1-6, more preferably 1-4, and
n is an integer from 5-20, preferably 8-12, more preferably 10,
k is an integer from 1-8, preferably 1-5, more preferably 1-3,
* indicates the bond closer to Cy, and
*** indicates the bond to D, preferably the bond to the nitrogen of a primary, secondary or tertiary amine group in D,
wherein AB is preferably bound to the remaining molecule via a thiol group of a cysteine residue.

14. A pharmaceutical composition comprising an antibody-drug conjugate according to claim 12 or 13 and a pharmaceutically acceptable carrier.

15. An antibody-drug conjugate according to 12 or 13 or a pharmaceutical composition according to claim 14 for use in medicine, particularly in human medicine.
